# EUROPEAN PATENT SPECIFICATION

(11) **EP 1 931 791 B1**
(45) Date of publication and mention of the grant of the patent: **08.02.2012**
(21) Application number: 06836107.0
(22) Date of filing: 22.08.2006
(51) Int. Cl.: C12N 15/82

(54) **METHODS AND COMPOSITIONS FOR THE EXPRESSION OF A POLYNUCLEOTIDE OF INTEREST**
VERFAHREN UND ZUSAMMENSETZUNGEN FÜR DEN AUSDRUCK EINES POLYNUKLEOTID VON INTERESSE
PROCEDES ET COMPOSITIONS POUR L'EXPRESSION D'UN POLYNUCLEOTIDE D'INTERET

(30) Priority: 24.08.2005 US 710854 P; 28.06.2006 US 817011 P
(43) Date of publication of application: 18.06.2008
(73) Proprietor: Pioneer-Hi-Bred International, Inc., Johnston, IA 50131-1014 (US); E.I. DU PONT DE NEMOURS AND COMPANY, Wilmington, DE 19898 (US)
(72) Inventor: MCCUTCHEN, Billy, Fred, College Station, TX 77845 (US); HAZEL, Christine, B., Port Deposit, MD 21904 (US); LIU, Donglong, Johnston, IA 50131 (US); LU, Albert, L., Newark, DE 19702 (US); MEHRE, Wayne, J., Urbandale, IA 50322 (US); OLSON, Paul, D., Kalaheo, HI 96741 (US); WONG, James, F. H., Johnson, IA 50131 (US)
(74) Representative: Bentham, Andrew
(86) International application number: PCT/US2006/032792
(87) International publication number: WO 2007/024866

(56) References cited:
- WO-A-02/20811
- US-A- 5 097 025
- US-A1- 2006 031 962
- US-B1- 6 603 062
- FANG R-X ET AL: "Multiple cis Regulatory Elements for Maximal Expression of the Cauliflower Mosaic Virus 35S Promoter in Transgenic Plants" PLANT CELL, AMERICAN SOCIETY OF PLANT PHYSIOLOGISTS, ROCKVILLE, MD, US, vol. 1, no. 1, January 1989 (1989-01), pages 141-150, XP002999683 ISSN: 1040-4651

### SEQUENCE LISTING

<110> McCutchen, Billy Fred
   Hazel, Christine B.
   Liu, Donglong
   Lu, Albert L.
   Mehre, Wayne J.
   Olson, Paul D.
   Wong, James F. H.
<120> Methods and Compositions for the Expression of a Polynucleotide of Interest
<130> 035718/315230
<150> 60/710,854
   <151> 2005-08-24
<150> 60/817,011
   <151> 2006-06-28
<160> 18
<170> FastSEQ for Windows Version 4.0
<210> 1
   <211> 438
   <212> DNA
   <213> cauliflower mosaic virus
<220>
   <221> enhancer
   <222> (0)...(0)
   <223> 35S enhancer
<400> 1
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgat                                               438
<210> 2
   <211> 534
   <212> DNA
   <213> cauliflower mosaic virus
<220>
   <221> promoter
   <222> (0) ... (0)
   <223> S35 enhancer with minimial core promoter
<400> 2
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgatat ctccactgac gtaagggatg acgcacaatc ccactaagct 480
   tcgcaagacc cttcctctat ataaggaagt tcatttcatt tggagaggac aggg       534
<210> 3
   <211> 52
   <212> DNA
   <213> cauliflower mosaic virus
<220>
   <221> promoter
   <222> (0)...(0)
   <223> mimimal core promoter
<400> 3
   gcaagaccct tcctctatat aaggaagttc atttcatttg gagaggacag gg 52
<210> 4
   <211> 162
   <212> DNA
   <213> cauliflower mosaic virus
<400> 4
   catcgttgaa gatgcctctg ccgacagtgg tcccaaagat ggacccccac ccacgaggag 60
   catcgtggaa aaagaagacg ttccaaccac gtcttcaaag caagtggatt gatgtgatat 120
   ctccactgac gtaagggatg acgcacaatc ccactaagct tc                    162
<210> 5
   <211> 44
   <212> DNA
   <213> cauliflower mosaic virus
<400> 5
   atctccactg acgtaaggga tgacgcacaa tcccactaag cttc 44
<210> 6
   <211> 1991
   <212> DNA
   <213> Artificial Sequence
<220>
   <223> promoter sequence comprising ZmUBI PRO-5'UTR-ZMUBI INTRON 1
<400> 6
   gcagtgcagc gtgacccggt cgtgcccctc tctagagata atgagcattg catgtctaag 60
   ttataaaaaa ttaccacata ttttttttgt cacacttgtt tgaagtgcag tttatctatc 120
   tttatacata tatttaaact ttactctacg aataatataa tctatagtac tacaataata 180
   tcagtgtttt agagaatcat ataaatgaac agttagacat ggtctaaagg acaattgagt 240
   attttgacaa caggactcta cagttttatc tttttagtgt gcatgtgttc tccttttttt 300
   ttgcaaatag cttcacctat ataatacttc atccatttta ttagtacatc catttagggt 360
   ttagggttaa tggtttttat agactaattt ttttagtaca tctattttat tctattttag 420
   cctctaaatt aagaaaacta aaactctatt ttagtttttt tatttaataa tttagatata 480
   aaatagaata aaataaagtg actaaaaatt aaacaaatac cctttaagaa attaaaaaaa 540
   ctaaggaaac atttttcttg tttcgagtag ataatgccag cctgttaaac gccgtcgacg 600
   agtctaacgg acaccaacca gcgaaccagc agcgtcgcgt cgggccaagc gaagcagacg 660
   gcacggcatc tctgtcgctg cctctggacc cctctcgaga gttccgctcc accgttggac 720
   ttgctccgct gtcggcatcc agaaattgcg tggcggagcg gcagacgtga gccggcacgg 780
   caggcggcct cctcctcctc tcacggcacc ggcagctacg ggggattcct ttcccaccgc 840
   tccttcgctt tcccttcctc gcccgccgta ataaatagac accccctcca caccctcttt 900
   ccccaacctc gtgttgttcg gagcgcacac acacacaacc agatctcccc caaatccacc 960
   cgtcggcacc tccgcttcaa ggtacgccgc tcgtcctccc ccccccccct ctctaccttc 1020
   tctagatcgg cgttccggtc catggttagg gcccggtagt tctacttctg ttcatgtttg 1080
   tgttagatcc gtgtttgtgt tagatccgtg ctgctagcgt tcgtacacgg atgcgacctg 1140
   tacgtcagac acgttctgat tgctaacttg ccagtgtttc tctttgggga atcctgggat 1200
   ggctctagcc gttccgcaga cgggatcgat ttcatgattt tttttgtttc gttgcatagg 1260
   gtttggtttg cccttttcct ttatttcaat atatgccgtg cacttgtttg tcgggtcatc 1320
   ttttcatgct tttttttgtc ttggttgtga tgatgtggtc tggttgggcg gtcgttctag 1380
   atcggagtag aattctgttt caaactacct ggtggattta ttaattttgg atctgtatgt 1440
   gtgtgccata catattcata gttacgaatt gaagatgatg gatggaaata tcgatctagg 1500
   ataggtatac atgttgatgc gggttttact gatgcatata cagagatgct ttttgttcgc 1560
   ttggttgtga tgatgtggtg tggttgggcg gtcgttcatt cgttctagat cggagtagaa 1620
   tactgtttca aactacctgg tgtatttatt aattttggaa ctgtatgtgt gtgtcataca 1680
   tcttcatagt tacgagttta agatggatgg aaatatcgat ctaggatagg tatacatgtt 1740
   gatgtgggtt ttactgatgc atatacatga tggcatatgc agcatctatt catatgctct 1800
   aaccttgagt acctatctat tataataaac aagtatgttt tataattatt ttgatcttga 1860
   tatacttgga tgatggcata tgcagcagct atatgtggat ttttttagcc ctgccttcat 1920
   acgctattta tttgcttggt actgtttctt ttgtcgatgc tcaccctgtt gtttggtgtt 1980
   acttctgcag g 1991
<210> 7
   <211> 441
   <212> DNA
   <213> Artificial Sequence
<220>
   <223> optimized GAT sequence (GAT4602)
<221> CDS
   <222> (1)...(441)
<400> 7
   atg ata gag gtg aaa ccg att aac gca gag gat acc tat gaa cta agg    48
   Met Ile Glu Val Lys Pro Ile Asn Ala Glu Asp Thr Tyr Glu Leu Arg
    1               5                  10                  15
   cat aga ata ctc aga cca aac cag ccg ata gaa gcg tgt atg ttt gaa    96
   His Arg Ile Leu Arg Pro Asn Gln Pro Ile Glu Ala Cys Met Phe Glu
               20                  25                  30
   agc gat tta ctt cgt ggt gca ttt cac tta ggc ggc tat tac ggg ggc   144
   Ser Asp Leu Leu Arg Gly Ala Phe His Leu Gly Gly Tyr Tyr Gly Gly
           35                  40                  45
   aaa ctg att tcc ata gct tca ttc cac cag gcc gag cac tca gaa ctc   192
   Lys Leu Ile Ser Ile Ala Ser Phe His Gln Ala Glu His Ser Glu Leu
       50                  55                  60
   caa ggc cag aaa cag tac cag ctc cga ggt atg gct acc ttg gaa ggt   240
   Gln Gly Gln Lys Gln Tyr Gln Leu Arg Gly Met Ala Thr Leu Glu Gly
    65                  70                  75                  80
   tat cgt gag cag aag gcg gga tcg agt cta att aaa cac gct gaa gaa   288
   Tyr Arg Glu Gln Lys Ala Gly Ser Ser Leu Ile Lys His Ala Glu Glu
                   85                  90                  95
   att ctt cgt aag agg ggg gcg gac ttg ctt tgg tgt aat gcg cgg aca   336
   Ile Leu Arg Lys Arg Gly Ala Asp Leu Leu Trp Cys Asn Ala Arg Thr
               100                 105                 110
   tcc gcc tca ggc tac tac aaa aag tta ggc ttc agc gag cag gga gag   384
   Ser Ala Ser Gly Tyr Tyr Lys Lys Leu Gly Phe Ser Glu Gln Gly Glu
           115                 120                 125
   gta ttc gac acg ccg cca gta gga cct cac atc ctg atg tat aaa agg   432
   Val Phe Asp Thr Pro Pro Val Gly Pro His Ile Leu Met Tyr Lys Arg
       130                 135                 140
   atc aca taa                                                       441
   Ile Thr  *
   145
<210> 8
   <211> 444
   <212> DNA
   <213> Artificial Sequence
<220>
   <223> Optimized GAT sequence -- GAT4621
<221> CDS
   <222> (1)...(444)
<400> 8
   atg gct att gag gtt aag cct atc aac gca gag gat acc tat gac ctt    48
   Met Ala Ile Glu Val Lys Pro Ile Asn Ala Glu Asp Thr Tyr Asp Leu
    1               5                  10                  15
   agg cat aga gtg ctc aga cca aac cag cct atc gaa gcc tgc atg ttt    96
   Arg His Arg Val Leu Arg Pro Asn Gln Pro Ile Glu Ala Cys Met Phe
               20                  25                  30
   gag tct gac ctt act agg agt gca ttt cac ctt ggt gga ttc tac gga    144
   Glu Ser Asp Leu Thr Arg Ser Ala Phe His Leu Gly Gly Phe Tyr Gly
           35                  40                  45
   ggt aaa ctg att tcc gtg gct tca ttc cac caa gct gag cac tct gaa    192
   Gly Lys Leu Ile Ser Val Ala Ser Phe His Gln Ala Glu His Ser Glu
       50                  55                  60
   ctt caa ggt aag aag cag tac cag ctt aga ggt gtg gct acc ttg gaa    240
   Leu Gln Gly Lys Lys Gln Tyr Gln Leu Arg Gly Val Ala Thr Leu Glu
    65                  70                  75                  80
   ggt tat aga gag cag aag gct ggt tcc agt ctc gtg aaa cac gct gaa    288
   Gly Tyr Arg Glu Gln Lys Ala Gly Ser Ser Leu Val Lys His Ala Glu
                    85                  90                  95
   gag att ctc aga aag aga ggt gct gac atg atc tgg tgt aat gcc agg    336
   Glu Ile Leu Arg Lys Arg Gly Ala Asp Met Ile Trp Cys Asn Ala Arg
               100                 105                 110
   aca tct gct tca gga tac tac agg aag ttg gga ttc agt gag caa gga    384
   Thr Ser Ala Ser Gly Tyr Tyr Arg Lys Leu Gly Phe Ser Glu Gln Gly
           115                 120                 125
   gag gtg ttc gat act cct cca gtt gga cct cac atc ctg atg tat aag    432
   Glu Val Phe Asp Thr Pro Pro Val Gly Pro His Ile Leu Met Tyr Lys
       130                 135                 140
   agg atc aca taa                                                    444
   Arg Ile Thr  *
   145
<210> 9
   <211> 3359
   <212> DNA
   <213> Artificial Sequence
<220>
   <223> 3X35S ENH operbably linked to the ZmUbi PRO-5UTR-ZmUbi intron 1 promoter ; 35S enhancer in the reverse direction
<400> 9
   atcacatcaa tccacttgct ttgaagacgt ggttggaacg tcttcttttt ccacgatgct 60
   cctcgtgggt gggggtccat ctttgggacc actgtcggca gaggcatctt caacgatggc 120
   ctttccttta tcgcaatgat ggcatttgta ggagccacct tccttttcca ctatcttcac 180
   aataaagtga cagatagctg ggcaatggaa tccgaggagg tttccggata ttaccctttg 240
   ttgaaaagtc tcaattgccc tttggtcttc tgagactgta tctttgatat ttttggagta 300
   gacaagcgtg tcgtgctcca ccatgttgac gaagattttc ttcttgtcat tgagtcgtaa 360
   gagactctgt atgaactgtt cgccagtctt tacggcgagt tctgttaggt cctctatttg 420
   aatctttgac tccatggacg gtatcgataa gctagcttga tatcacatca atccacttgc 480
   tttgaagacg tggttggaac gtcttctttt tccacgatgc tcctcgtggg tgggggtcca 540
   tctttgggac cactgtcggc agaggcatct tcaacgatgg cctttccttt atcgcaatga 600
   tggcatttgt aggagccacc ttccttttcc actatcttca caataaagtg acagatagct 660
   gggcaatgga atccgaggag gtttccggat attacccttt gttgaaaagt ctcaattgcc 720
   ctttggtctt ctgagactgt atctttgata tttttggagt agacaagcgt gtcgtgctcc 780
   accatgttga cgaagatttt cttcttgtca ttgagtcgta agagactctg tatgaactgt 840
   tcgccagtct ttacggcgag ttctgttagg tcctctattt gaatctttga ctccatgatc 900
   gaattatcac atcaatccac ttgctttgaa gacgtggttg gaacgtcttc tttttccacg 960
   atgctcctcg tgggtggggg tccatctttg ggaccactgt cggcagaggc atcttcaacg 1020
   atggcctttc ctttatcgca atgatggcat ttgtaggagc caccttcctt ttccactatc 1080
   ttcacaataa agtgacagat agctgggcaa tggaatccga ggaggtttcc ggatattacc 1140
   ctttgttgaa aagtctcaat tgccctttgg tcttctgaga ctgtatcttt gatatttttg 1200
   gagtagacaa gcgtgtcgtg ctccaccatg ttgacgaaga ttttcttctt gtcattgagt 1260
   cgtaagagac tctgtatgaa ctgttcgcca gtctttacgg cgagttctgt taggtcctct 1320
   atttgaatct ttgactccat gggaattcct gcagcccagc ttgcatgcct gcagtgcagc 1380
   gtgacccggt cgtgcccctc tctagagata atgagcattg catgtctaag ttataaaaaa 1440
   ttaccacata ttttttttgt cacacttgtt tgaagtgcag tttatctatc tttatacata 1500
   tatttaaact ttactctacg aataatataa tctatagtac tacaataata tcagtgtttt 1560
   agagaatcat ataaatgaac agttagacat ggtctaaagg acaattgagt attttgacaa 1620
   caggactcta cagttttatc tttttagtgt gcatgtgttc tccttttttt ttgcaaatag 1680
   cttcacctat ataatacttc atccatttta ttagtacatc catttagggt ttagggttaa 1740
   tggtttttat agactaattt ttttagtaca tctattttat tctattttag cctctaaatt 1800
   aagaaaacta aaactctatt ttagtttttt tatttaataa tttagatata aaatagaata 1860
   aaataaagtg actaaaaatt aaacaaatac cctttaagaa attaaaaaaa ctaaggaaac 1920
   atttttcttg tttcgagtag ataatgccag cctgttaaac gccgtcgacg agtctaacgg 1980
   acaccaacca gcgaaccagc agcgtcgcgt cgggccaagc gaagcagacg gcacggcatc 2040
   tctgtcgctg cctctggacc cctctcgaga gttccgctcc accgttggac ttgctccgct 2100
   gtcggcatcc agaaattgcg tggcggagcg gcagacgtga gccggcacgg caggcggcct 2160
   cctcctcctc tcacggcacc ggcagctacg ggggattcct ttcccaccgc tccttcgctt 2220
   tcccttcctc gcccgccgta ataaatagac accccctcca caccctcttt ccccaacctc 2280
   gtgttgttcg gagcgcacac acacacaacc agatctcccc caaatccacc cgtcggcacc 2340
   tccgcttcaa ggtacgccgc tcgtcctccc ccccccccct ctctaccttc tctagatcgg 2400
   cgttccggtc catggttagg gcccggtagt tctacttctg ttcatgtttg tgttagatcc 2460
   gtgtttgtgt tagatccgtg ctgctagcgt tcgtacacgg atgcgacctg tacgtcagac 2520
   acgttctgat tgctaacttg ccagtgtttc tctttgggga atcctgggat ggctctagcc 2580
   gttccgcaga cgggatcgat ttcatgattt tttttgtttc gttgcatagg gtttggtttg 2640
   cccttttcct ttatttcaat atatgccgtg cacttgtttg tcgggtcatc ttttcatgct 2700
   tttttttgtc ttggttgtga tgatgtggtc tggttgggcg gtcgttctag atcggagtag 2760
   aattctgttt caaactacct ggtggattta ttaattttgg atctgtatgt gtgtgccata 2820
   catattcata gttacgaatt gaagatgatg gatggaaata tcgatctagg ataggtatac 2880
   atgttgatgc gggttttact gatgcatata cagagatgct ttttgttcgc ttggttgtga 2940
   tgatgtggtg tggttgggcg gtcgttcatt cgttctagat cggagtagaa tactgtttca 3000
   aactacctgg tgtatttatt aattttggaa ctgtatgtgt gtgtcataca tcttcatagt 3060
   tacgagttta agatggatgg aaatatcgat ctaggatagg tatacatgtt gatgtgggtt 3120
   ttactgatgc atatacatga tggcatatgc agcatctatt catatgctct aaccttgagt 3180
   acctatctat tataataaac aagtatgttt tataattatt ttgatcttga tatacttgga 3240
   tgatggcata tgcagcagct atatgtggat ttttttagcc ctgccttcat acgctattta 3300
   tttgcttggt actgtttctt ttgtcgatgc tcaccctgtt gtttggtgtt acttctgca  3359
<210> 10
   <211> 1343
   <212> DNA
   <213> cauliflower mosaic virus
<220>
   <221> enhancer
   <222> (0)...(0)
   <223> 35S ehancer 3X in reverse direction
<400> 10
   atcacatcaa tccacttgct ttgaagacgt ggttggaacg tcttcttttt ccacgatgct 60
   cctcgtgggt gggggtccat ctttgggacc actgtcggca gaggcatctt caacgatggc 120
   ctttccttta tcgcaatgat ggcatttgta ggagccacct tccttttcca ctatcttcac 180
   aataaagtga cagatagctg ggcaatggaa tccgaggagg tttccggata ttaccctttg 240
   ttgaaaagtc tcaattgccc tttggtcttc tgagactgta tctttgatat ttttggagta 300
   gacaagcgtg tcgtgctcca ccatgttgac gaagattttc ttcttgtcat tgagtcgtaa 360
   gagactctgt atgaactgtt cgccagtctt tacggcgagt tctgttaggt cctctatttg 420
   aatctttgac tccatggacg gtatcgataa gctagcttga tatcacatca atccacttgc 480
   tttgaagacg tggttggaac gtcttctttt tccacgatgc tcctcgtggg tgggggtcca 540
   tctttgggac cactgtcggc agaggcatct tcaacgatgg cctttccttt atcgcaatga 600
   tggcatttgt aggagccacc ttccttttcc actatcttca caataaagtg acagatagct 660
   gggcaatgga atccgaggag gtttccggat attacccttt gttgaaaagt ctcaattgcc 720
   ctttggtctt ctgagactgt atctttgata tttttggagt agacaagcgt gtcgtgctcc 780
   accatgttga cgaagatttt cttcttgtca ttgagtcgta agagactctg tatgaactgt 840
   tcgccagtct ttacggcgag ttctgttagg tcctctattt gaatctttga ctccatgatc 900
   gaattatcac atcaatccac ttgctttgaa gacgtggttg gaacgtcttc tttttccacg 960
   atgctcctcg tgggtggggg tccatctttg ggaccactgt cggcagaggc atcttcaacg 1020
   atggcctttc ctttatcgca atgatggcat ttgtaggagc caccttcctt ttccactatc 1080
   ttcacaataa agtgacagat agctgggcaa tggaatccga ggaggtttcc ggatattacc 1140
   ctttgttgaa aagtctcaat tgccctttgg tcttctgaga ctgtatcttt gatatttttg 1200
   gagtagacaa gcgtgtcgtg ctccaccatg ttgacgaaga ttttcttctt gtcattgagt 1260
   cgtaagagac tctgtatgaa ctgttcgcca gtctttacgg cgagttctgt taggtcctct 1320
   atttgaatct ttgactccat ggg                                         1343
<210> 11
   <211> 2479
   <212> DNA
   <213> Artificial Sequence
<220>
   <223> 35S ENH(+):ZmUBI PRO-5UTR-UBI INTRON1 ; 35S enhancer in the forward direction
<400> 11
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgatat caagcttatc gataccgtcg acctcgaggg ggggcccagc 480
   ttgcatgcct gcagtgcagc gtgacccggt cgtgcccctc tctagagata atgagcattg 540
   catgtctaag ttataaaaaa ttaccacata ttttttttgt cacacttgtt tgaagtgcag 600
   tttatctatc tttatacata tatttaaact ttactctacg aataatataa tctatagtac 660
   tacaataata tcagtgtttt agagaatcat ataaatgaac agttagacat ggtctaaagg 720
   acaattgagt attttgacaa caggactcta cagttttatc tttttagtgt gcatgtgttc 780
   tccttttttt ttgcaaatag cttcacctat ataatacttc atccatttta ttagtacatc 840
   catttagggt ttagggttaa tggtttttat agactaattt ttttagtaca tctattttat 900
   tctattttag cctctaaatt aagaaaacta aaactctatt ttagtttttt tatttaataa 960
   tttagatata aaatagaata aaataaagtg actaaaaatt aaacaaatac cctttaagaa 1020
   attaaaaaaa ctaaggaaac atttttcttg tttcgagtag ataatgccag cctgttaaac 1080
   gccgtcgacg agtctaacgg acaccaacca gcgaaccagc agcgtcgcgt cgggccaagc 1140
   gaagcagacg gcacggcatc tctgtcgctg cctctggacc cctctcgaga gttccgctcc 1200
   accgttggac ttgctccgct gtcggcatcc agaaattgcg tggcggagcg gcagacgtga 1260
   gccggcacgg caggcggcct cctcctcctc tcacggcacc ggcagctacg ggggattcct 1320
   ttcccaccgc tccttcgctt tcccttcctc gcccgccgta ataaatagac accccctcca 1380
   caccctcttt ccccaacctc gtgttgttcg gagcgcacac acacacaacc agatctcccc 1440
   caaatccacc cgtcggcacc tccgcttcaa ggtacgccgc tcgtcctccc ccccccccct 1500
   ctctaccttc tctagatcgg cgttccggtc catggttagg gcccggtagt tctacttctg 1560
   ttcatgtttg tgttagatcc gtgtttgtgt tagatccgtg ctgctagcgt tcgtacacgg 1620
   atgcgacctg tacgtcagac acgttctgat tgctaacttg ccagtgtttc tctttgggga 1680
   atcctgggat ggctctagcc gttccgcaga cgggatcgat ttcatgattt tttttgtttc 1740
   gttgcatagg gtttggtttg cccttttcct ttatttcaat atatgccgtg cacttgtttg 1800
   tcgggtcatc ttttcatgct tttttttgtc ttggttgtga tgatgtggtc tggttgggcg 1860
   gtcgttctag atcggagtag aattctgttt caaactacct ggtggattta ttaattttgg 1920
   atctgtatgt gtgtgccata catattcata gttacgaatt gaagatgatg gatggaaata 1980
   tcgatctagg ataggtatac atgttgatgc gggttttact gatgcatata cagagatgct 2040
   ttttgttcgc ttggttgtga tgatgtggtg tggttgggcg gtcgttcatt cgttctagat 2100
   cggagtagaa tactgtttca aactacctgg tgtatttatt aattttggaa ctgtatgtgt 2160
   gtgtcataca tcttcatagt tacgagttta agatggatgg aaatatcgat ctaggatagg 2220
   tatacatgtt gatgtgggtt ttactgatgc atatacatga tggcatatgc agcatctatt 2280
   catatgctct aaccttgagt acctatctat tataataaac aagtatgttt tataattatt 2340
   ttgatcttga tatacttgga tgatggcata tgcagcagct atatgtggat ttttttagcc 2400
   ctgccttcat acgctattta tttgcttggt actgtttctt ttgtcgatgc tcaccctgtt 2460
   gtttggtgtt acttctgca                                               2479
<210> 12
   <211> 3331
   <212> DNA
   <213> Artificial Sequence
<220>
   <223> 3X35S ENH (+):ZmUBI PRO-5UTR-UBI INTRON1; 35S enhancer in the forward direction
<400> 12
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgataa ttcgatcatg gagtcaaaga ttcaaataga ggacctaaca 480
   gaactcgccg taaagactgg cgaacagttc atacagagtc tcttacgact caatgacaag 540
   aagaaaatct tcgtcaacat ggtggagcac gacacgcttg tctactccaa aaatatcaaa 600
   gatacagtct cagaagacca aagggcaatt gagacttttc aacaaagggt aatatccgga 660
   aacctcctcg gattccattg cccagctatc tgtcacttta ttgtgaagat agtggaaaag 720
   gaaggtggct cctacaaatg ccatcattgc gataaaggaa aggccatcgt tgaagatgcc 780
   tctgccgaca gtggtcccaa agatggaccc ccacccacga ggagcatcgt ggaaaaagaa 840
   gacgttccaa ccacgtcttc aaagcaagtg gattgatgtg atatcaagct tatcgatacc 900
   gccatggagt caaagattca aatagaggac ctaacagaac tcgccgtaaa gactggcgaa 960
   cagttcatac agagtctctt acgactcaat gacaagaaga aaatcttcgt caacatggtg 1020
   gagcacgaca cgcttgtcta ctccaaaaat atcaaagata cagtctcaga agaccaaagg 1080
   gcaattgaga cttttcaaca aagggtaata tccggaaacc tcctcggatt ccattgccca 1140
   gctatctgtc actttattgt gaagatagtg gaaaaggaag gtggctccta caaatgccat 1200
   cattgcgata aaggaaaggc catcgttgaa gatgcctctg ccgacagtgg tcccaaagat 1260
   ggacccccac ccacgaggag catcgtggaa aaagaagacg ttccaaccac gtcttcaaag 1320
   caagtggatt gatgtgatgt ctgcagtgca gcgtgacccg gtcgtgcccc tctctagaga 1380
   taatgagcat tgcatgtcta agttataaaa aattaccaca tatttttttt gtcacacttg 1440
   tttgaagtgc agtttatcta tctttataca tatatttaaa ctttactcta cgaataatat 1500
   aatctatagt actacaataa tatcagtgtt ttagagaatc atataaatga acagttagac 1560
   atggtctaaa ggacaattga gtattttgac aacaggactc tacagtttta tctttttagt 1620
   gtgcatgtgt tctccttttt ttttgcaaat agcttcacct atataatact tcatccattt 1680
   tattagtaca tccatttagg gtttagggtt aatggttttt atagactaat ttttttagta 1740
   catctatttt attctatttt agcctctaaa ttaagaaaac taaaactcta ttttagtttt 1800
   tttatttaat aatttagata taaaatagaa taaaataaag tgactaaaaa ttaaacaaat 1860
   accctttaag aaattaaaaa aactaaggaa acatttttct tgtttcgagt agataatgcc 1920
   agcctgttaa acgccgtcga cgagtctaac ggacaccaac cagcgaacca gcagcgtcgc 1980
   gtcgggccaa gcgaagcaga cggcacggca tctctgtcgc tgcctctgga cccctctcga 2040
   gagttccgct ccaccgttgg acttgctccg ctgtcggcat ccagaaattg cgtggcggag 2100
   cggcagacgt gagccggcac ggcaggcggc ctcctcctcc tctcacggca ccggcagcta 2160
   cgggggattc ctttcccacc gctccttcgc tttcccttcc tcgcccgccg taataaatag 2220
   acaccccctc cacaccctct ttccccaacc tcgtgttgtt cggagcgcac acacacacaa 2280
   ccagatctcc cccaaatcca cccgtcggca cctccgcttc aaggtacgcc gctcgtcctc 2340
   cccccccccc ctctctacct tctctagatc ggcgttccgg tccatggtta gggcccggta 2400
   gttctacttc tgttcatgtt tgtgttagat ccgtgtttgt gttagatccg tgctgctagc 2460
   gttcgtacac ggatgcgacc tgtacgtcag acacgttctg attgctaact tgccagtgtt 2520
   tctctttggg gaatcctggg atggctctag ccgttccgca gacgggatcg atttcatgat 2580
   tttttttgtt tcgttgcata gggtttggtt tgcccttttc ctttatttca atatatgccg 2640
   tgcacttgtt tgtcgggtca tcttttcatg cttttttttg tcttggttgt gatgatgtgg 2700
   tctggttggg cggtcgttct agatcggagt agaattctgt ttcaaactac ctggtggatt 2760
   tattaatttt ggatctgtat gtgtgtgcca tacatattca tagttacgaa ttgaagatga 2820
   tggatggaaa tatcgatcta ggataggtat acatgttgat gcgggtttta ctgatgcata 2880
   tacagagatg ctttttgttc gcttggttgt gatgatgtgg tgtggttggg cggtcgttca 2940
   ttcgttctag atcggagtag aatactgttt caaactacct ggtgtattta ttaattttgg 3000
   aactgtatgt gtgtgtcata catcttcata gttacgagtt taagatggat ggaaatatcg 3060
   atctaggata ggtatacatg ttgatgtggg ttttactgat gcatatacat gatggcatat 3120
   gcagcatcta ttcatatgct ctaaccttga gtacctatct attataataa acaagtatgt 3180
   tttataatta ttttgatctt gatatacttg gatgatggca tatgcagcag ctatatgtgg 3240
   atttttttag ccctgccttc atacgctatt tatttgcttg gtactgtttc ttttgtcgat 3300
   gctcaccctg ttgtttggtg ttacttctgc a                                3331
<210> 13
   <211> 2454
   <212> DNA
   <213> Artificial Sequence
<220>
   <223> 35S ENH (-):ZmUBI PRO-5UTR-UBI INTRON1 ; 35S enhancer in the reverse direction
<400> 13
   atcacatcaa tccacttgct ttgaagacgt ggttggaacg tcttcttttt ccacgatgct 60
   cctcgtgggt gggggtccat ctttgggacc actgtcggca gaggcatctt caacgatggc 120
   ctttccttta tcgcaatgat ggcatttgta ggagccacct tccttttcca ctatcttcac 180
   aataaagtga cagatagctg ggcaatggaa tccgaggagg tttccggata ttaccctttg 240
   ttgaaaagtc tcaattgccc tttggtcttc tgagactgta tctttgatat ttttggagta 300
   gacaagcgtg tcgtgctcca ccatgttgac gaagattttc ttcttgtcat tgagtcgtaa 360
   gagactctgt atgaactgtt cgccagtctt tacggcgagt tctgttaggt cctctatttg 420
   aatctttgac tccatgggaa ttcctgcagc ccagcttgca tgcctgcagt gcagcgtgac 480
   ccggtcgtgc ccctctctag agataatgag cattgcatgt ctaagttata aaaaattacc 540
   acatattttt tttgtcacac ttgtttgaag tgcagtttat ctatctttat acatatattt 600
   aaactttact ctacgaataa tataatctat agtactacaa taatatcagt gttttagaga 660
   atcatataaa tgaacagtta gacatggtct aaaggacaat tgagtatttt gacaacagga 720
   ctctacagtt ttatcttttt agtgtgcatg tgttctcctt tttttttgca aatagcttca 780
   cctatataat acttcatcca ttttattagt acatccattt agggtttagg gttaatggtt 840
   tttatagact aattttttta gtacatctat tttattctat tttagcctct aaattaagaa 900
   aactaaaact ctattttagt ttttttattt aataatttag atataaaata gaataaaata 960
   aagtgactaa aaattaaaca aatacccttt aagaaattaa aaaaactaag gaaacatttt 1020
   tcttgtttcg agtagataat gccagcctgt taaacgccgt cgacgagtct aacggacacc 1080
   aaccagcgaa ccagcagcgt cgcgtcgggc caagcgaagc agacggcacg gcatctctgt 1140
   cgctgcctct ggacccctct cgagagttcc gctccaccgt tggacttgct ccgctgtcgg 1200
   catccagaaa ttgcgtggcg gagcggcaga cgtgagccgg cacggcaggc ggcctcctcc 1260
   tcctctcacg gcaccggcag ctacggggga ttcctttccc accgctcctt cgctttccct 1320
   tcctcgcccg ccgtaataaa tagacacccc ctccacaccc tctttcccca acctcgtgtt 1380
   gttcggagcg cacacacaca caaccagatc tcccccaaat ccacccgtcg gcacctccgc 1440
   ttcaaggtac gccgctcgtc ctcccccccc cccctctcta ccttctctag atcggcgttc 1500
   cggtccatgg ttagggcccg gtagttctac ttctgttcat gtttgtgtta gatccgtgtt 1560
   tgtgttagat ccgtgctgct agcgttcgta cacggatgcg acctgtacgt cagacacgtt 1620
   ctgattgcta acttgccagt gtttctcttt ggggaatcct gggatggctc tagccgttcc 1680
   gcagacggga tcgatttcat gatttttttt gtttcgttgc atagggtttg gtttgccctt 1740
   ttcctttatt tcaatatatg ccgtgcactt gtttgtcggg tcatcttttc atgctttttt 1800
   ttgtcttggt tgtgatgatg tggtctggtt gggcggtcgt tctagatcgg agtagaattc 1860
   tgtttcaaac tacctggtgg atttattaat tttggatctg tatgtgtgtg ccatacatat 1920
   tcatagttac gaattgaaga tgatggatgg aaatatcgat ctaggatagg tatacatgtt 1980
   gatgcgggtt ttactgatgc atatacagag atgctttttg ttcgcttggt tgtgatgatg 2040
   tggtgtggtt gggcggtcgt tcattcgttc tagatcggag tagaatactg tttcaaacta 2100
   cctggtgtat ttattaattt tggaactgta tgtgtgtgtc atacatcttc atagttacga 2160
   gtttaagatg gatggaaata tcgatctagg ataggtatac atgttgatgt gggttttact 2220
   gatgcatata catgatggca tatgcagcat ctattcatat gctctaacct tgagtaccta 2280
   tctattataa taaacaagta tgttttataa ttattttgat cttgatatac ttggatgatg 2340
   gcatatgcag cagctatatg tggatttttt tagccctgcc ttcatacgct atttatttgc 2400
   ttggtactgt ttcttttgtc gatgctcacc ctgttgtttg gtgttacttc tgca       2454
<210> 14
   <211> 3359
   <212> DNA
   <213> Artificial Sequence
<220>
   <223> 3X35S ENH (-): ZmUBI PRO-5UTR-UBI INTRON1 ; 35S enhancer in the reverse direction
<400> 14
   atcacatcaa tccacttgct ttgaagacgt ggttggaacg tcttcttttt ccacgatgct 60
   cctcgtgggt gggggtccat ctttgggacc actgtcggca gaggcatctt caacgatggc 120
   ctttccttta tcgcaatgat ggcatttgta ggagccacct tccttttcca ctatcttcac 180
   aataaagtga cagatagctg ggcaatggaa tccgaggagg tttccggata ttaccctttg 240
   ttgaaaagtc tcaattgccc tttggtcttc tgagactgta tctttgatat ttttggagta 300
   gacaagcgtg tcgtgctcca ccatgttgac gaagattttc ttcttgtcat tgagtcgtaa 360
   gagactctgt atgaactgtt cgccagtctt tacggcgagt tctgttaggt cctctatttg 420
   aatctttgac tccatggacg gtatcgataa gctagcttga tatcacatca atccacttgc 480
   tttgaagacg tggttggaac gtcttctttt tccacgatgc tcctcgtggg tgggggtcca 540
   tctttgggac cactgtcggc agaggcatct tcaacgatgg cctttccttt atcgcaatga 600
   tggcatttgt aggagccacc ttccttttcc actatcttca caataaagtg acagatagct 660
   gggcaatgga atccgaggag gtttccggat attacccttt gttgaaaagt ctcaattgcc 720
   ctttggtctt ctgagactgt atctttgata tttttggagt agacaagcgt gtcgtgctcc 780
   accatgttga cgaagatttt cttcttgtca ttgagtcgta agagactctg tatgaactgt 840
   tcgccagtct ttacggcgag ttctgttagg tcctctattt gaatctttga ctccatgatc 900
   gaattatcac atcaatccac ttgctttgaa gacgtggttg gaacgtcttc tttttccacg 960
   atgctcctcg tgggtggggg tccatctttg ggaccactgt cggcagaggc atcttcaacg 1020
   atggcctttc ctttatcgca atgatggcat ttgtaggagc caccttcctt ttccactatc 1080
   ttcacaataa agtgacagat agctgggcaa tggaatccga ggaggtttcc ggatattacc 1140
   ctttgttgaa aagtctcaat tgccctttgg tcttctgaga ctgtatcttt gatatttttg 1200
   gagtagacaa gcgtgtcgtg ctccaccatg ttgacgaaga ttttcttctt gtcattgagt 1260
   cgtaagagac tctgtatgaa ctgttcgcca gtctttacgg cgagttctgt taggtcctct 1320
   atttgaatct ttgactccat gggaattcct gcagcccagc ttgcatgcct gcagtgcagc 1380
   gtgacccggt cgtgcccctc tctagagata atgagcattg catgtctaag ttataaaaaa 1440
   ttaccacata ttttttttgt cacacttgtt tgaagtgcag tttatctatc tttatacata 1500
   tatttaaact ttactctacg aataatataa tctatagtac tacaataata tcagtgtttt 1560
   agagaatcat ataaatgaac agttagacat ggtctaaagg acaattgagt attttgacaa 1620
   caggactcta cagttttatc tttttagtgt gcatgtgttc tccttttttt ttgcaaatag 1680
   cttcacctat ataatacttc atccatttta ttagtacatc catttagggt ttagggttaa 1740
   tggtttttat agactaattt ttttagtaca tctattttat tctattttag cctctaaatt 1800
   aagaaaacta aaactctatt ttagtttttt tatttaataa tttagatata aaatagaata 1860
   aaataaagtg actaaaaatt aaacaaatac cctttaagaa attaaaaaaa ctaaggaaac 1920
   atttttcttg tttcgagtag ataatgccag cctgttaaac gccgtcgacg agtctaacgg 1980
   acaccaacca gcgaaccagc agcgtcgcgt cgggccaagc gaagcagacg gcacggcatc 2040
   tctgtcgctg cctctggacc cctctcgaga gttccgctcc accgttggac ttgctccgct 2100
   gtcggcatcc agaaattgcg tggcggagcg gcagacgtga gccggcacgg caggcggcct 2160
   cctcctcctc tcacggcacc ggcagctacg ggggattcct ttcccaccgc tccttcgctt 2220
   tcccttcctc gcccgccgta ataaatagac accccctcca caccctcttt ccccaacctc 2280
   gtgttgttcg gagcgcacac acacacaacc agatctcccc caaatccacc cgtcggcacc 2340
   tccgcttcaa ggtacgccgc tcgtcctccc ccccccccct ctctaccttc tctagatcgg 2400
   cgttccggtc catggttagg gcccggtagt tctacttctg ttcatgtttg tgttagatcc 2460
   gtgtttgtgt tagatccgtg ctgctagcgt tcgtacacgg atgcgacctg tacgtcagac 2520
   acgttctgat tgctaacttg ccagtgtttc tctttgggga atcctgggat ggctctagcc 2580
   gttccgcaga cgggatcgat ttcatgattt tttttgtttc gttgcatagg gtttggtttg 2640
   cccttttcct ttatttcaat atatgccgtg cacttgtttg tcgggtcatc ttttcatgct 2700
   tttttttgtc ttggttgtga tgatgtggtc tggttgggcg gtcgttctag atcggagtag 2760
   aattctgttt caaactacct ggtggattta ttaattttgg atctgtatgt gtgtgccata 2820
   catattcata gttacgaatt gaagatgatg gatggaaata tcgatctagg ataggtatac 2880
   atgttgatgc gggttttact gatgcatata cagagatgct ttttgttcgc ttggttgtga 2940
   tgatgtggtg tggttgggcg gtcgttcatt cgttctagat cggagtagaa tactgtttca 3000
   aactacctgg tgtatttatt aattttggaa ctgtatgtgt gtgtcataca tcttcatagt 3060
   tacgagttta agatggatgg aaatatcgat ctaggatagg tatacatgtt gatgtgggtt 3120
   ttactgatgc atatacatga tggcatatgc agcatctatt catatgctct aaccttgagt 3180
   acctatctat tataataaac aagtatgttt tataattatt ttgatcttga tatacttgga 3240
   tgatggcata tgcagcagct atatgtggat ttttttagcc ctgccttcat acgctattta 3300
   tttgcttggt actgtttctt ttgtcgatgc tcaccctgtt gtttggtgtt acttctgca  3359
<210> 15
   <211> 1340
   <212> DNA
   <213> Artificial Sequence
<220>
   <223> 3X35S enhancer in the forward direction
<400> 15
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgataa ttcgatcatg gagtcaaaga ttcaaataga ggacctaaca 480
   gaactcgccg taaagactgg cgaacagttc atacagagtc tcttacgact caatgacaag 540
   aagaaaatct tcgtcaacat ggtggagcac gacacgcttg tctactccaa aaatatcaaa 600
   gatacagtct cagaagacca aagggcaatt gagacttttc aacaaagggt aatatccgga 660
   aacctcctcg gattccattg cccagctatc tgtcacttta ttgtgaagat agtggaaaag 720
   gaaggtggct cctacaaatg ccatcattgc gataaaggaa aggccatcgt tgaagatgcc 780
   tctgccgaca gtggtcccaa agatggaccc ccacccacga ggagcatcgt ggaaaaagaa 840
   gacgttccaa ccacgtcttc aaagcaagtg gattgatgtg atatcaagct tatcgatacc 900
   gccatggagt caaagattca aatagaggac ctaacagaac tcgccgtaaa gactggcgaa 960
   cagttcatac agagtctctt acgactcaat gacaagaaga aaatcttcgt caacatggtg 1020
   gagcacgaca cgcttgtcta ctccaaaaat atcaaagata cagtctcaga agaccaaagg 1080
   gcaattgaga cttttcaaca aagggtaata tccggaaacc tcctcggatt ccattgccca 1140
   gctatctgtc actttattgt gaagatagtg gaaaaggaag gtggctccta caaatgccat 1200
   cattgcgata aaggaaaggc catcgttgaa gatgcctctg ccgacagtgg tcccaaagat 1260
   ggacccccac ccacgaggag catcgtggaa aaagaagacg ttccaaccac gtcttcaaag 1320
   caagtggatt gatgtgatgt                                             1340
<210> 16
   <211> 438
   <212> DNA
   <213> cauliflower mosaic virus
<220>
   <221> misc_feature
   <222> (0)...(0)
   <223> 1X 35S enhancer in reverse direction
<400> 16
   atcacatcaa tccacttgct ttgaagacgt ggttggaacg tcttcttttt ccacgatgct 60
   cctcgtgggt gggggtccat ctttgggacc actgtcggca gaggcatctt caacgatggc 120
   ctttccttta tcgcaatgat ggcatttgta ggagccacct tccttttcca ctatcttcac 180
   aataaagtga cagatagctg ggcaatggaa tccgaggagg tttccggata ttaccctttg 240
   ttgaaaagtc tcaattgccc tttggtcttc tgagactgta tctttgatat ttttggagta 300
   gacaagcgtg tcgtgctcca ccatgttgac gaagattttc ttcttgtcat tgagtcgtaa 360
   gagactctgt atgaactgtt cgccagtctt tacggcgagt tctgttaggt cctctatttg 420
   aatctttgac tccatggg                                               438
<210> 17
   <211> 1344
   <212> DNA
   <213> Artificial Sequence
<220>
   <223> 3X 35S enhancer in forward direction
<400> 17
   cccatggagt caaagattca aatagaggac ctaacagaac tcgccgtaaa gactggcgaa 60
   cagttcatac agagtctctt acgactcaat gacaagaaga aaatcttcgt caacatggtg 120
   gagcacgaca cgcttgtcta ctccaaaaat atcaaagata cagtctcaga agaccaaagg 180
   gcaattgaga cttttcaaca aagggtaata tccggaaacc tcctcggatt ccattgccca 240
   gctatctgtc actttattgt gaagatagtg gaaaaggaag gtggctccta caaatgccat 300
   cattgcgata aaggaaaggc catcgttgaa gatgcctctg ccgacagtgg tcccaaagat 360
   ggacccccac ccacgaggag catcgtggaa aaagaagacg ttccaaccac gtcttcaaag 420
   caagtggatt gatgtgataa ttcgatcatg gagtcaaaga ttcaaataga ggacctaaca 480
   gaactcgccg taaagactgg cgaacagttc atacagagtc tcttacgact caatgacaag 540
   aagaaaatct tcgtcaacat ggtggagcac gacacgcttg tctactccaa aaatatcaaa 600
   gatacagtct cagaagacca aagggcaatt gagacttttc aacaaagggt aatatccgga 660
   aacctcctcg gattccattg cccagctatc tgtcacttta ttgtgaagat agtggaaaag 720
   gaaggtggct cctacaaatg ccatcattgc gataaaggaa aggccatcgt tgaagatgcc 780
   tctgccgaca gtggtcccaa agatggaccc ccacccacga ggagcatcgt ggaaaaagaa 840
   gacgttccaa ccacgtcttc aaagcaagtg gattgatgtg atatcaagct agcttatcga 900
   taccgtccat ggagtcaaag attcaaatag aggacctaac agaactcgcc gtaaagactg 960
   gcgaacagtt catacagagt ctcttacgac tcaatgacaa gaagaaaatc ttcgtcaaca 1020
   tggtggagca cgacacgctt gtctactcca aaaatatcaa agatacagtc tcagaagacc 1080
   aaagggcaat tgagactttt caacaaaggg taatatccgg aaacctcctc ggattccatt 1140
   gcccagctat ctgtcacttt attgtgaaga tagtggaaaa ggaaggtggc tcctacaaat 1200
   gccatcattg cgataaagga aaggccatcg ttgaagatgc ctctgccgac agtggtccca 1260
   aagatggacc cccacccacg aggagcatcg tggaaaaaga agacgttcca accacgtctt 1320
   caaagcaagt ggattgatgt gatg                                        1344
<210> 18
   <211> 1344
   <212> DNA
   <213> Artificial Sequence
<220>
   <223> 3X 35S enhancer-in reverse direction
<400> 18
   catcacatca atccacttgc tttgaagacg tggttggaac gtcttctttt tccacgatgc 60
   tcctcgtggg tgggggtcca tctttgggac cactgtcggc agaggcatct tcaacgatgg 120
   cctttccttt atcgcaatga tggcatttgt aggagccacc ttccttttcc actatcttca 180
   caataaagtg acagatagct gggcaatgga atccgaggag gtttccggat attacccttt 240
   gttgaaaagt ctcaattgcc ctttggtctt ctgagactgt atctttgata tttttggagt 300
   agacaagcgt gtcgtgctcc accatgttga cgaagatttt cttcttgtca ttgagtcgta 360
   agagactctg tatgaactgt tcgccagtct ttacggcgag ttctgttagg tcctctattt 420
   gaatctttga ctccatggac ggtatcgata agctagcttg atatcacatc aatccacttg 480
   ctttgaagac gtggttggaa cgtcttcttt ttccacgatg ctcctcgtgg gtgggggtcc 540
   atctttggga ccactgtcgg cagaggcatc ttcaacgatg gcctttcctt tatcgcaatg 600
   atggcatttg taggagccac cttccttttc cactatcttc acaataaagt gacagatagc 660
   tgggcaatgg aatccgagga ggtttccgga tattaccctt tgttgaaaag tctcaattgc 720
   cctttggtct tctgagactg tatctttgat atttttggag tagacaagcg tgtcgtgctc 780
   caccatgttg acgaagattt tcttcttgtc attgagtcgt aagagactct gtatgaactg 840
   ttcgccagtc tttacggcga gttctgttag gtcctctatt tgaatctttg actccatgat 900
   cgaattatca catcaatcca cttgctttga agacgtggtt ggaacgtctt ctttttccac 960
   gatgctcctc gtgggtgggg gtccatcttt gggaccactg tcggcagagg catcttcaac 1020
   gatggccttt cctttatcgc aatgatggca tttgtaggag ccaccttcct tttccactat 1080
   cttcacaata aagtgacaga tagctgggca atggaatccg aggaggtttc cggatattac 1140
   cctttgttga aaagtctcaa ttgccctttg gtcttctgag actgtatctt tgatattttt 1200
   ggagtagaca agcgtgtcgt gctccaccat gttgacgaag attttcttct tgtcattgag 1260
   tcgtaagaga ctctgtatga actgttcgcc agtctttacg gcgagttctg ttaggtcctc 1320
   tatttgaatc tttgactcca tggg                                        1344

## Description

### FIELD OF THE INVENTION

The present invention is drawn to the field of genetics and molecular biology. More particularly, the compositions and methods are directed to expression of polynucleotides of interest.

### BACKGROUND OF THE INVENTION

Expression of heterologous DNA sequences in a plant host is dependent upon the presence of an operably linked promoter that is functional within the plant host.

Choice of the promoter sequence will determine when and where within the organism the heterologous DNA sequence is expressed. Where expression in specific tissues or organs is desired, tissue-preferred promoters may be used. Where gene expression in response to a stimulus is desired, inducible promoters are the regulatory element of choice. In contrast, where continuous expression is desired throughout the cells of a plant, constitutive promoters are utilized. Additional regulatory sequences upstream and/or downstream from the core promoter sequence may be included in the expression constructs of transformation vectors to bring about varying levels of expression of heterologous nucleotide sequences in a transgenic plant.

Frequently it is desirable to express a DNA sequence in particular tissues or organs of a plant For example, increased resistance of a plant to infection by soil-and air-borne pathogens might be accomplished by genetic manipulation of the plant's genome to comprise a tissue-preferred promoter operably linked to a heterologous pathogen-resistance gene such that pathogen-resistance proteins are produced in the desired plant tissue.

Alternatively, it might be desirable to inhibit expression of a native DNA sequence within a plant's tissues to achieve a desired phenotype. In this case, such inhibition might be accomplished with transformation of the plant to comprise a tissue-preferred promoter operably linked to an antisense nucleotide sequence, such that expression of the antisense sequence produces an RNA transcript that interferes with translation of the mRNA of the native DNA sequence.

Thus, isolation and characterization of regulatory sequences that can be positioned upstream and/or downstream from the core promoter sequence and allow varying levels of expression of heterologous nucleotide sequences in a transgenic plant are needed for genetic manipulation of plants.

### BRIEF SUMMARY OF THE INVENTION

The invention provides a method of expressing a polynucleotide of interest comprising introducing into a monocot plant cell at least one DNA construct comprising a chimeric transcriptional regulatory region comprising at least one enhancer domain operably linked to a heterologous promoter, said chimeric transcriptional regulatory region operably linked to the polynucleotide of interest, wherein said enhancer domain is selected from:
(a) the nucleotide sequence comprising SEQ ID NO: 17 or at least 3 copies of SEQ ID NO:1, wherein said enhancer domain is in the reverse orientation and operably linked to said promoter;
(b) the nucleotide sequence comprising at least 95% sequence identity to SEQ ID NO: 17, wherein said nucleotide sequence has transcriptional regulatory activity and wherein said enhancer domain is in the reverse orientation and operably linked to said promoter; or,
(c) a nucleotide sequence comprising at least 3 copies of a sequence having 95% sequence identity to SEQ ID NO: 1, wherein said nucleotide sequence retains transcriptional regulatory activity, wherein said enhancer domains are in the reverse orientation and operably linked to said promoter.

In a preferred embodiment said enhancer domain does not comprise the sequence set forth in SEQ ID NO:5. In another preferred embodiment said copies of said enhancer are immediately adjacent to one another.

In another preferred embodiment said polynucleotide of interest encodes a polypeptide; and/or a selectable marker. In a more preferred embodiment said selectable marker comprises a polynucleotide that confers tolerance to a herbicide. In an even more preferred embodiment said method further comprises culturing said monocot plant cell in a media comprising an effective concentration of the herbicide.

The invention further provides a method to select a monocot plant cell having a polynucleotide of interest comprising:
a) providing a population of monocot plant cells;
b) introducing into at least one of the monocot plant cell from said population a DNA construct comprising the polynucleotide of interest and further comprising a chimeric transcriptional regulatory region comprising at least one enhancer domain operably linked to a first heterologous promoter, said chimeric transcriptional regulatory region operably linked to a selectable marker, wherein said enhancer domain is selected from:
   i) the nucleotide sequence comprising SEQ ID NO: 17 or at least 3 copies of SEQ ID NO:1, and wherein said enhancer domain is in the reverse orientation and operably linked to said promoter;
   ii) the nucleotide sequence comprising at least 95% sequence identity to SEQ ID NO:17, wherein said nucleotide sequence has transcriptional regulatory activity, wherein said enhancer domain is in the reverse orientation and operably linked to said promoter; and,
   iii) a nucleotide sequence comprises at least 3 copies of a sequence having 95% sequence identity to SEQ ID NO: 1, wherein said nucleotide sequence retains transcriptional regulatory activity, and wherein said enhancer domains are in the reverse orientation and operably linked to said promoter; and,
c) contacting said population of the monocot plant cells with an effective concentration of an appropriate selection agent; and,
d) selecting the monocot plant cell expressing said selectable marker, and thereby identifying a monocot plant cell having the polynucleotide of interest.

In a preferred embodiment said enhancer domain does not comprise the sequence set forth in SEQ ID NO: 5. In another preferred embodiment the copies of said enhancer are immediately adjacent to one another. In another preferred embodiment said selectable marker encodes a polypeptide that confers tolerance to a herbicide. In a more preferred embodiment said selective agent comprises a herbicide. In another preferred embodiment said DNA construct comprises in the 5' to 3' or 3' to 5' orientation: the first polynucleotide of interest operably linked to a second heterologous promoter, operably linked to at least one of said enhancer domains, operably linked to the first heterologous promoter, operably linked to the selectable marker, wherein said first polynucleotide of interest and said selectable marker are expressed in divergent directions.

The invention further provides a polynucleotide comprising a chimeric transcriptional regulatory element comprising a promoter that drives expression in a cell operably linked to at least one copy of a heterologous enhancer domain wherein said enhancer domain is selected from:
(a) the nucleotide sequence comprising SEQ ID NO:17 or at least 3 copies of SEQ ID NO:1 wherein said enhancer domain is in the reverse orientation and operably linked to said promoter;
(b) the nucleotide sequence comprising at least 95% sequence identity to SEQ ID NO: 17, wherein said nucleotide sequence has regulating transcriptional activity, and is in the reverse orientation and operably linked to said promoter; or,
(c) a nucleotide sequence comprises at least 3 copies of a sequence having 95% sequence identity to SEQ ID NO: 1, wherein said nucleotide sequence retains transcriptional regulatory activity, wherein said enhancer domains are in the reverse orientation and operably linked to said promoter.

In a preferred embodiment said enhancer domain does not comprise the sequence of SEQ ID NO: 5. In another preferred embodiment the copies of said enhancer are immediately adjacent to one another.

The invention further provides an expression vector, monocot plant cell or monocot plant comprising the polynucleotide of the invention.

In a preferred embodiment the methods of the invention increase single copy integration event in the monocot plant cell.

### BRIEF DESCRIPTION OF THE DRAWING(S)

Figure 1 provides examples of constructs having 35S enhancer elements.
Figure 2 provides a schematic demonstrating the effect of 35S enhancers on TX efficiency.
Figure 3 provides a schematic demonstrating the effect of 35S enhancers on T0 efficiency.
Figure 4 provides a schematic demonstrating the effect of 35S enhancers on event copy number.
Figure 5 provides a schematic demonstrating the effect of 35S enhancers on expression.
Figure 6 provides an insecticidal gene evaluation assay.
Figure 7 provides a schematic showing the development of a GAT selection scheme.

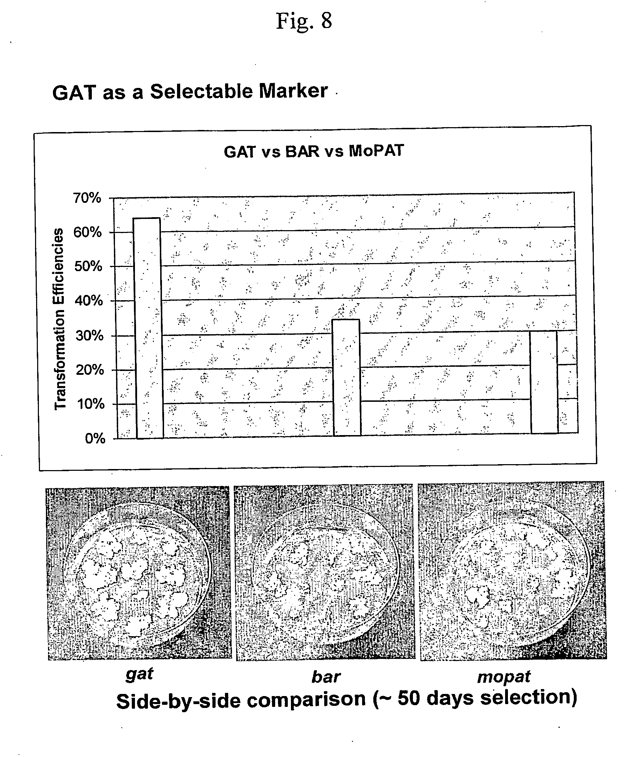

Figure 8 demonstrates that GAT can be used as a selectable marker.
Figure 9 provides a schematic demonstrating GAT transformation efficiencies.

### DETAILED DESCRIPTION OF THE INVENTION

The present inventions now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

### I. Polynucleotides

Compositions of the invention comprise an enhancer domain of a transcription control region and variants and fragments of that enhancer domain. When the enhancer domain is operably linked to a promoter, a functional transcriptional regulation region is formed which can direct expression of an operably linked polynucleotide of interest. In particular, the present invention provides isolated polynucleotides comprising the enhancer domain set forth in SEQ ID NO: 17 or at least three copies of SEQ ID NO:1 active variants and fragments thereof; and, polynucleotide consisting of the sequence of SEQ ID NO: 17 or at least three copies of SEQ ID NO:1. In still further embodiments, the enhancer domain employed does not comprise the region to the 35S promoter from about position -90 to about -46. The -90 to -46 region of the promoter is set forth in SEQ ID NO:5.

The term "promoter" is intended to mean a regulatory region of DNA comprising a transcriptional initiation region, which in some embodiments, comprises a TATA box capable of directing RNA polymerase II to initiate RNA synthesis at the appropriate transcription initiation site for a particular coding sequence. The promoter can further be operably linked to additional regulatory elements that influence transcription, including, but not limited to, introns, 5' untranslated regions, and enhancer elements. As used herein, an "enhancer sequence," "enhancer domain," "enhancer element," or "enhancer," when operably linked to an appropriate promoter, will modulate the level of transcription of an operably linked polynucleotide of interest. In specific embodiments, the enhancer of the invention can alter normal promoter expression patterns. For example, a tissue preferred/specific promoter, when operably linked to an enhancer of the invention, will demonstrate ectopic expression that is not normally observed with the promoter alone. For example, the oleosin promoter is a seed-preferred promoter but shows leaf expression in the presence of the enhancer of the invention or active variant or fragment thereof. In another example, the Zrp2 promoter, which is a leaf-preferred promoter, becomes constitutive when operably linked to the enhancer sequence of the invention or a biologically active variant or fragment thereof, and the RM2 promoter, a root promoter, when operably linked to the enhancer of the invention or biologically active variant or fragment thereof will show leaf expression. Thus, the compositions of the present invention further comprise a polynucleotide comprising a chimeric transcriptional control region comprising the promoter operably linked to at least one, two, three, four or more copies of the enhancer domain or an active variant or fragment of the domain. Such compositions allow for the expression of an operably linked polynucleotide of interest.

The invention also relates to isolated or substantially purified polynucleotide or protein compositions. An "isolated" or "purified" polynucleotide, or biologically active portion thereof, is substantially or essentially free from components that normally accompany or interact with the polynucleotide as found in its naturally occurring environment. Thus, an isolated or purified polynucleotide is substantially free of other cellular material, or culture medium when produced by recombinant techniques, or substantially free of chemical precursors or other chemicals when chemically synthesized. Optimally, an "isolated" polynucleotide is free of sequences (optimally protein encoding sequences) that naturally flank the polynucleotide (i.e., sequences located at the 5' and 3' ends of the polynucleotide) in the genomic DNA of the organism from which the polynucleotide is derived. For example, in various embodiments, the isolated polynucleotide can contain less than about 5 kb, 4 kb, 3 kb, 2 kb, 1kb, 0.5 kb, or 0.1 kb of nucleotide sequence that naturally flank the polynucleotide in genomic DNA of the cell from which the polynucleotide is derived. A polynucleotide that is substantially free of cellular material includes preparations of having less than about 30%, 20%, 10%, 5%, or 1% (by dry weight) of contaminating protein or polynucleotides. When the polynucleotide of the invention or biologically active portion thereof is recombinantly produced, optimally culture medium represents less than about 30%, 20%, 10%, 5%, or 1% (by dry weight) of chemical precursors.

Fragments and variants of the disclosed enhancer sequence are also encompassed by the present invention. In particular, variants of the enhancer domain of SEQ ID NO: 17 is provided.

In specific embodiments, the active variants of the enhancer comprise conserved regions. Such regions include one or more of the underlined sequences set forth below

See, also, Fang et al. (1989) The Plant Cell 1:141-150. Methods to detect transcriptional regulation include, for example, assaying for the level of the operably linked polynucleotide of interest or assaying for the expression of a polypeptide encoded by an operably linked sequence of interest. Such assays can directly measure the level of the polynucleotide or polypeptide or they can assay for the activity or an expected phenotype when the expression of the sequence is altered. Such assays are known in the art. As used herein, the term "variants" means substantially similar sequences. For polynucleotides, naturally occurring variants can be identified with the use of

well-known molecular biology techniques, such as, for example, with polymerase chain reaction (PCR) and hybridization techniques as outlined herein. For polynucleotides, a variant comprises a deletion and/or addition of one or more nucleotides at one or more internal sites within the native polynucleotide and/or a substitution of one or more nucleotides at one or more sites in the native polynucleotide. As used herein, a "native" polynucleotides comprises a naturally occurring nucleotide sequence. For polynucleotides, naturally occurring variants can be identified with the use of well-known molecular biology techniques, as, for example, with polymerase chain reaction (PCR) and hybridization techniques as outlined below. Variant polynucleotides also include synthetically derived nucleotide sequences, such as those generated, for example, by using site-directed mutagenesis. Generally, variants of a particular polynucleotides of the invention will have at least about 95%, 96%, 97%, 98%, 99% or more sequence identity to that particular polynucleotides as determined by sequence alignment programs and parameters described elsewhere herein. A biologically active variant of a polynucleotides of the invention may differ from that sequence by as few as 1-15 nucleic acid residues, as few as 1-10, such as 6-10, as few as 10, 9, 8, 7, 6, 5, 4, 3, 2, or even 1 nucleic acid residue.

Thus, the genes and polynucleotides of the invention include both the naturally occurring sequences as well as mutant forms. Such variants will continue to possess the desired transcription regulatory activity. The deletions, insertions, and substitutions of the sequences encompassed herein are not expected to produce radical changes in the characteristics of the sequence. However, when it is difficult to predict the exact effect of the substitution, deletion, or insertion in advance of doing so, one skilled in the art will appreciate that the effect will be evaluated by routine screening assays. That is, the activity can be evaluated using assays disclosed elsewhere herein.

Variant polynucleotides also encompass sequences derived from a mutagenic and recombinogenic procedure such as DNA shuffling. With such a procedure, one or more different enhancer domain sequences for the promoter can be manipulated to create a new enhancer domain. In this manner, libraries of recombinant polynucleotides are generated from a population of related sequence polynucleotides comprising sequence regions that have substantial sequence identity and can be homologously recombined *in vitro* or *in vivo.* Strategies for such DNA shuffling are known in the art. See, for example, Stemmer (1994) Proc. Natl. Acad Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad Sci. USA 94:4504-4509; Crameri et al. (1998) Nature 391:288-291; and U.S. Patent Nos. 5,605,793 and 5,837,458.

The polynucleotides of the invention can be used to isolate corresponding sequences from other organisms, particularly other plants, more particularly other monocots. In this manner, methods such as PCR, hybridization, and the like can be used to identify such sequences based on their sequence homology to the sequences set forth herein. Sequences isolated based on their sequence identity to the entire enhancer domain set forth herein or to fragments or variants thereof are encompassed by the present invention.

In a PCR approach, oligonucleotide primers can be designed for use in PCR reactions to amplify corresponding DNA sequences from genomic DNA extracted from any plant of interest. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York), hereinafter Sambrook. See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

In hybridization techniques, all or part of a known polynucleotide is used as a probe that selectively hybridizes to other corresponding nucleotide sequences present in a population of cloned genomic DNA fragments from a chosen organism. The hybridization probes may be labeled with a detectable group such as ³²P, or any other detectable marker. Thus, for example, probes for hybridization can be made by labeling synthetic oligonucleotides based on the enhancer domain of the invention. Methods for preparation of probes for hybridization and for construction of genomic libraries are generally known in the art and are disclosed in Sambrook.

For example, the entire enhancer domain sequence disclosed herein, or one or more portions thereof, may be used as a probe capable of specifically hybridizing to corresponding enhancer sequences. To achieve specific hybridization under a variety of conditions, such probes include sequences that are unique among enhancer domain sequences and are at least about 10 nucleotides in length or at least about 20 nucleotides in length. Such probes may be used to amplify corresponding 35S enhancer sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Cloning: A Laboratory Manual (2nd ed, Cold Spring Harbor Laboratory Press, Plainview, New York.)

Hybridization of such sequences may be carried out under stringent conditions. The terms "stringent conditions" and "stringent hybridization conditions" are intended to mean conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length or less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a final wash in 0.1X SSC at 60 to 65°C for a duration of at least 30 minutes. Duration of hybridization is generally less than about 24 hours, usually about 4 to about 12 hours. The duration of the wash time will be at least a length of time sufficient to reach equilibrium.

Specificity is typically the function of post-hybridization washes, the critical factors being the ionic strength and temperature of the final wash solution. For DNA-DNA hybrids, the Tₘ (thermal melting point) can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the Tₘ for the specific sequence and its complement at a defined ionic strength and pH. However, severely stringent conditions can utilize a hybridization and/or wash at 1, 2, 3, or 4°C lower than the Tₘ; moderately stringent conditions can utilize a hybridization and/or wash at 6, 7, 8, 9, or 10°C lower than the Tₘ; low stringency conditions can utilize a hybridization and/or wash at 11, 12, 13, 14, 15, or 20°C lower than the Tₘ. Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See also Sambrook.

Thus, isolated sequences that have embryo-preferred promoter activity and which hybridize under stringent conditions to the enhancer domain sequences disclosed herein, or to fragments thereof, are encompassed by the present invention. Generally, stringent conditions are selected to be about 5°C lower than the Tₘ for the specific sequence at a defined ionic strength and pH. However, stringent conditions encompass temperatures in the range of about 1°C to about 20°C lower than the Tₘ, depending upon the desired degree of stringency as otherwise qualified herein.

The following terms are used to describe the sequence relationships between two or more polynucleotides or polypeptides: (a) "reference sequence", (b) "comparison window", (c) "sequence identity", and, (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two polynucleotides. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well known in the art. Thus, the determination of percent sequence identity between any two sequences can be accomplished using a mathematical algorithm. Non-limiting examples of such mathematical algorithms are the algorithm of Myers and Miller (1988) CABIOS 4:11-17; the local alignment algorithm of Smith et al. (1981) Adv. Appl. Math. 2:482; the global alignment algorithm of Needleman and Wunsch (1970) J. Mol. Biol. 48:443-453; the search-for-local alignment method of Pearson and Lipman (1988) Proc. Natl. Acad. Sci. 85:2444-2448; the algorithm of Karlin and Altschul (1990) Proc. Natl. Acad. Sci. USA 872264, modified as in Karlin and Altschul (1993) Proc. Natl. Acad. Sci. USA 90:5873-5877.

Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA). Alignments using these programs can be performed using the default parameters. The CLUSTAL program is well described by Higgins et al. (1988) Gene 73:237-244 (1988); Higgins et al. (1989) CABIOS 5:151-153; Corpet et al. (1988) Nucleic Acids Res. 16:10881-90; Huang et al. (1992) CABIOS 8:155-65; and Pearson et al. (1994) Meth. Mol. Biol. 24:307-331. The ALIGN program is based on the algorithm of Myers and Miller (1988) *supra.* A PAM120 weight residue table, a gap length penalty of 12, and a gap penalty of 4 can be used with the ALIGN program when comparing amino acid sequences. The BLAST programs of Altschul et al (1990) J. Mol. Biol. 215:403 are based on the algorithm of Karlin and Altschul (1990) *supra.* BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul *et al.* (1997) *supra.* When utilizing BLAST, Gapped BLAST, PSI-BLAST, the default parameters of the respective programs (e.g., BLASTN for nucleotide sequences, BLASTX for proteins) can be used. See www.ncbi.nlm.nih.gov. Alignment may also be performed manually by inspection.

Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna. cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.

GAP uses the algorithm of Needleman and Wunsch (1970) J. Mol. Biol. 48:443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the GCG Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10, 15, 20, 25, 30, 35, 40, 45, 50, 55, 60, 65 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the GCG Wisconsin Genetics Software Package is BLOSUM62 (see Henikoff and Henikoff (1989) Proc. Natl. Acad. Sci. USA 89:10915).

(c) As used herein, "sequence identity" or "identity" in the context of two polynucleotides or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).

(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

### II. DNA Constructs

The use of the term "polynucleotide" is not intended to limit the present invention to polynucleotides comprising DNA. Those of ordinary skill in the art will recognize that polynucleotides, can comprise ribonucleotides and combinations of ribonucleotides and deoxyribonucleotides. Such deoxyribonucleotides and ribonucleotides includes both naturally occurring molecules and synthetic analogues. The polynucleotides of the invention also encompass all forms of sequences including, but not limited to, single-stranded forms, double-stranded forms, hairpins, stem-and-loop structures, and the like.

The polynucleotide disclosed in the present invention, as well as, variants thereof, are useful in the genetic manipulation of any plant. The enhancer domain or the active variant thereof are useful in this aspect when operably linked to a heterologous promoter. Such transcriptional regulatory control regions are referred to herein as "chimeric" transcriptional regulatory control regions. The chimeric transcriptional control regions can be opererably linked to a polynucleotide whose expression is to be controlled to achieve a desired phenotypic response. In this manner, the polynucleotide for the enhancer domain or the chimeric transcriptional regulatory control region of the invention may be provided in expression cassettes along with a polynucleotide of interest for expression in the organism of interest.

The chimeric transcriptional regulatory region can comprise at least 3 copies of the enhancer domain of SEQ ID NO:1. In specific embodiments, the chimeric transcriptional regulatory control region comprises at least 3, 4, 5, 6, 7 or more copies of the enhancer domain of SEQ ID NO:1. In further embodiments, the enhancer domain employed does not comprise the sequence set forth in SEQ ID NO:5.

The distance between the promoter and the enhancer domain can vary, so long as the chimeric transcriptional regulatory region continues to direct transcription of the operably linked polynucleotide of interest in the desired manner. For example, an enhancer domain can be positioned at least about 10000 to about 15000, about 10000 to about a 9000, about 9000 to about 8000, about 8000 to about 7000, about 7000 to about 6000, about 6000 to about 5000, about 5000 to about 4000, about 4000 to about 3000, about 3000 to about 2000, about 2000 to about 1000, about 1000 to about 500, about 500 to about 250, about 250 to immediately adjacent to the promoter. It is further recognized that one or more copies of the enhancer can be placed upstream (5') of the prompter or alternatively, one or more copies of the enhancer can be located 3' to the promoter. In specific embodiments, when located 3' of the promoter, the enhancer is downstream of the terminator region. In still further embodiments, said enhancers are arranged in the 3' to 5' orientation.

If multiple enhancers are employed, the enhancers can be positioned in the construct with respect to the promoter such that the desired affect on expression is achieved. For example, the enhances can be immediately adjacent to each other or at least between 1 to 100, 100 to 300, 300 to 500, 500 to 1000 nucleotides apart.

Such constructs can be provided in expression cassettes for expression in the organism of interest. The cassette can include 3' regulatory sequences and 5' regulatory sequences operably linked to a polynucleotide of interest "Operably linked" is intended to mean a functional linkage between two or more elements. For example, an operable linkage between a polynucleotide of interest and a regulatory sequence (i.e., a promoter) is a functional link that allows for expression of the polynucleotide of interest. An operable linkage between an enhancer and a transcriptional initiation region is a linkage which allows for the transcriptional control region to transcribe an operably linked polynucleotide of interest. Operably linked elements may be contiguous or non-contiguous. When used to refer to the joining of two protein coding regions, by operably linked is intended that the coding regions are in the same reading frame. The cassette may additionally contain at least one additional gene to be cotransformed into the organism. Alternatively, the additional gene(s) can be provided on multiple expression cassettes. Such an expression cassette is provided with a plurality of restriction sites and/or recombination sites for insertion of the polynucleotide of interest to be under the transcriptional regulation of the regulatory regions. The expression cassette may additionally contain selectable marker genes.

The expression cassette can include, in the 5'-3' direction of transcription, a chimeric transcriptional regulatory region comprising the enhancer domain or an active variant or fragment thereof, a promoter, or active variant or fragment thereof, a translational initiation region, a polynucleotide of interest, a translational termination region and, optionally, a transcriptional termination region functional in the host organism. The regulatory regions (i.e., promoters, enhancer domains, and translational termination regions, etc.) and/or the polynucleotide of interest may be native/analogous to the host cell or to each other. Alternatively, the regulatory regions and/or the polynucleotide of interest may be heterologous to the host cell or to each other.

As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. A "heterologous polynucleotide" as it relates to an enhancer domain is intended to mean a sequence that is not naturally occurring with the enhancer domain sequence of the invention. While this promoter is heterologous to the enhancer domain sequence, it may be homologous, or native, or heterologous, or foreign, to the plant host.

The termination region may be native with the enhancer domain or the transcriptional initiation region, may be native with the operably linked polynucleotide of interest, may be native with the plant host, or may be derived from another source (i.e., foreign or heterologous) to the enhancer domain, the transcriptional initiation region, , the polynucleotide of interest, the plant host, or any combination thereof. Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

The expression cassette comprising the sequences of the present invention may also contain at least one additional nucleotide sequence for a gene to be cotransformed into the organism. Alternatively, the additional sequence(s) can be provided on another expression cassette.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The expression cassettes may additionally contain 5' leader sequences. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) (Virology 154:9-20), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

The expression cassette can also comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). Additional selectable markers include phenotypic markers such as β-galactosidase and fluorescent proteins such as green fluorescent protein (GFP) (Su et al. (2004) Biotechnol Bioeng 85: 610-9 and Fetter et al. (2004) Plant Cell 16:215-28), cyan florescent protein (CYP) (Bolte et al. (2004) J. Cell Science 117:943-54 and Kato et al. (2002) Plant Physiol 129:913-42), and yellow florescent protein (PhiYFP™ from Evrogen, see, Bolte et al. (2004) J. Cell Science 117:943-54). For additional selectable markers, see generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Aci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724. The above list of selectable marker genes is not meant to be limiting. Any selectable marker gene can be used in the present invention.

As discussed above, a chimeric transcriptional regulatory region comprising SEQ ID NO: 17; a nucleotide sequence comprising at least 95% identity to SEQ ID NO: 17 and has transcriptional regulatory activity; or at least three copies of the enhancer domain of SEQ ID NO: 1 or active variant thereof operably linked to a heterologous promoter are provided. Any promoter of interest can be operably linked to the enhancer domain of the invention. Such promoters can be selected based on the desired outcome and can comprises constitutive, inducible, tissue-preferred, or other promoters for expression in a host cell of interest (i.e., a plant). Constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

In one embodiment, the promoter comprises an inducible promoter, particularly from a pathogen-inducible promoter. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116. See also WO 99/43819.

Promoters that are expressed locally at or near the site of pathogen infection can be used. See, for example, Marineau et al. (1987) Plant Mol. Biol. 9:335-342; Matton et al. (1989) Molecular Plant-Microbe Interactions 2:325-331; Somsisch et al. (1986) Proc. Natl. Acad Sci. USA 83:2427-2430; Somsisch et al. (1988) Mol. Gen. Genet. 2:93-98; and Yang (1996) Proc. Natl. Acad. Sci. USA 93:14972-14977. See also, Chen et al. (1996) Plant J. 10:955-966; Zhang et al. (1994) Proc. Natl. Acad. Sci. USA 91:2507-2511; Warner et al. (1993) Plant J. 3:191-201; Siebertz et al. (1989) Plant Cell 1:961-968; U.S. Patent No. 5,750,386 (nematode-inducible); and the references cited therein. Of particular interest is the inducible promoter for the maize PRms gene, whose expression is induced by the pathogen *Fusarium moniliforme* (see, for example, Cordero et al. (1992) Physiol. Mol. Plant Path. 41:189-200).

Additionally, as pathogens find entry into plants through wounds or insect damage, a wound-inducible promoter may be used in the constructions of the invention. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, U.S. Patent No. 5,428,148; win1 and win2 (Stanford et al. (1989) Mol. Gen. Genet. 215:200-208); systemin (McGurl et al. (1992) Science 225:1570-1573); WIP1 (Rohmeier et al. (1993) Plant Mol. Biol. 22:783-792; Eckelkamp et al. (1993) FEBS Letters 323:73-76); MPI gene (Corderok et al. (1994) Plant J. 6(2):141-150); and the like.

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Tissue-preferred promoters can be utilized to target enhanced expression of a polynucleotide of interest within a particular plant tissue. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

Leaf-preferred promoters are known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

Root-preferred promoters are known and can be selected from the many available from the literature or isolated de novo from various compatible species. See, for example, Hire et al. (1992) Plant Mol. Biol. 20(2):207-218 (soybean root-specific glutamine synthetase gene); Keller and Baumgartner (1991) Plant Cell 3(10):1051-1061 (root-specific control element in the GRP 1.8 gene of French bean); Sanger et al. (1990) Plant Mol. Biol. 14(3):433-443 (root-specific promoter of the mannopine synthase (MAS) gene of *Agrobacterium tumefaciens*); and Miao et al. (1991) Plant Cell 3(1):11-22 (full-length cDNA clone encoding cytosolic glutamine synthetase (GS), which is expressed in roots and root nodules of soybean). See also Bogusz et al. (1990) Plant Cell 2(7):633-641, where two root-specific promoters isolated from hemoglobin genes from the nitrogen-fixing nonlegume *Parasponia andersonii* and the related non-nitrogen-fixing nonlegume *Trema tomentosa* are described. The promoters of these genes were linked to a β-glucuronidase reporter gene and introduced into both the nonlegume *Nicotiana tabacum* and the legume *Lotus corniculatus,* and in both instances root-specific promoter activity was preserved. Leach and Aoyagi (1991) describe their analysis of the promoters of the highly expressed rolC and rolD root-inducing genes of *Agrobacterium rhizogenes* (see Plant Science (Limerick) 79(1):69-76). They concluded that enhancer and tissue-preferred DNA determinants are dissociated in those promoters. Teeri *et al.* (1989) used gene fusion to lacZ to show that the *Agrobacterium* T-DNA. gene encoding octopine synthase is especially active in the epidermis of the root tip and that the TR2' gene is root specific in the intact plant and stimulated by wounding in leaf tissue, an especially desirable combination of characteristics for use with an insecticidal or larvicidal gene (see EMBO J. 8(2):343-350). The TR1' gene, fused to *nptII* (neomycin phosphotransferase II) showed similar characteristics. Additional root-preferred promoters include the VfENOD-GRP3 gene promoter (Kuster et al. (1995) Plant Mol. Biol. 29(4):759-772); and rolB promoter (Capana et al. (1994) Plant Mol. Biol. 25(4):681-691. See also U.S. Patent Nos. 5,837,876; 5,750,386; 5,633,363; 5,459,252; 5,401,836; 5,110,732; and 5,023,179.

Seed-preferred promoters include both seed-specific promoters (those promoters active during seed development such as promoters of seed storage proteins) as well as seed-germinating promoters (those promoters active during seed germination). See Thompson et al. (1989) Bio Essays 10:108, herein incorporated by reference. Such seed-preferred promoters include, but are not limited to, Cim1 (cytokinin-induced message); cZ19B1 (maize 19 kDa zein); milps (myo-inositol-1-phosphate synthase) (see WO 00/11177 and U.S. Patent No. 6,225,529; herein incorporated by reference). Gamma-zein is an endosperm-specific promoter. Globulin 1 (Glb-1) is a representative embryo-specific promoter. For dicots, seed-specific promoters include, but are not limited to, bean β-phaseolin, napin, β-conglycinin, soybean lectin, cruciferin, and the like. For monocots, seed-specific promoters include, but are not limited to, maize 15 kDa zein, 22 kDa zein, 27 kDa zein, gamma-zein, waxy, shrunken 1, shrunken 2, Globulin 1, etc. See also WO 00/12733, where seed-preferred promoters from *end1* and *end2* genes are disclosed.

Polynucleotide sequences expressed by the chimeric transcriptional regulatory control region of the invention may be used for varying the phenotype of a plant. Various changes in phenotype are of interest including modifying expression of a gene in a plant embryo, altering a plant's pathogen or insect defense mechanism, increasing the plants tolerance to herbicides in a plant, altering embryo development to respond to environmental stress, and the like. These results can be achieved by the expression of a heterologous nucleotide sequence of interest comprising an appropriate gene product. In specific embodiments, the heterologous nucleotide sequence of interest is an endogenous plant sequence whose expression level is increased in the plant or plant part Alternatively, the results can be achieved by providing for a reduction of expression of one or more polynucleotide of interest.

The chimeric transcriptional regulatory control region of the invention can further comprise additional portions of other transcriptional regulatory regions. Thus, the transcriptional regulatory control element disclosed herein, comprising the enhancer domain and a heterologous promoter can comprise upstream regulatory elements such as, those responsible for tissue and temporal expression of the coding sequence. In the context of this disclosure, the term "regulatory element" also refers to a sequence of DNA, usually, but not always, upstream (5') to the coding sequence of a structural gene, which includes sequences which modulate the expression of the coding region. It is to be understood that nucleotide sequences, located within introns, or 3' of the coding region sequence may also contribute to the regulation of expression of a coding region of interest. Examples of suitable introns include, but are not limited to, the maize IVS6 intron, or the maize actin intron. A regulatory element may also include those elements located downstream (3') to the site of transcription initiation, or within transcribed regions, or both. In the context of the present invention a post-transcriptional regulatory element may include elements that are active following transcription initiation, for example translational and transcriptional enhancers, translational and transcriptional repressors, and mRNA stability determinants.

It is further recognized that the enhancer of the invention can be positioned in the DNA construct between and operably linked to a first and a second promoter. In such embodiments, the enhancer allows for a modulation in expression of both the first and the second promoters from a divergent direction. Exemplary, but non-limiting, examples of such DNA constructs comprise in the 5' to 3' or 3' to 5' orientation: a first polynucleotide of interest operably linked to a first promoter, operably linked to at least one copy of an enhancer of the invention, operably linked to a second promoter, operably linked to a second polynucleotide of interest. In specific embodiments, the enhancer sequence is heterologous to the first and the second enhancer sequence.

### III. Plants and Parts Thereof

As used herein, the term plant includes plant cells, plant protoplasts, plant cell tissue cultures from which plants can be regenerated, plant calli, plant clumps, and plant cells that are intact in plants or parts of plants such as embryos, pollen ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species. Progeny, variants, and mutants of the regenerated plants are also included within the scope of the invention, provided that these parts comprise the introduced polynucleotides.

The present invention may be used for transformation of any monocot plant species. Examples of plant species of interest include, but are not limited to, corn (*Zea mays*), rice (*Oryza sativa*)*,* rye (*Secale cereale*)*,* sorghum (*Sorghum bicolor, Sorghum vulgare*)*,* millet (e.g., pearl millet (*Pennisetum glaucum*)*,* proso millet (*Panicum miliaceum*)*,* foxtail millet (*Setaria italica*)*,* finger millet (*Eleusine coracana*)), wheat (*Triticum aestivum*), coconut (*Cocos nucifera*)*,* pineapple (*Ananas comosus*)*,* banana (*Musa* spp.), papaya (*Carica papaya*)*,* sugarcane (*Saccharum* spp.), oats, barley and daffodils (Narcissus spp).
In specific embodiments, plants of the present invention are monocot crop plants (for example, corn, sorghum, wheat, millet, etc.). In other embodiments, corn plants are optimal.

Other plants of interest include monocot grain plants that provide seeds of interest, oil-seed plants. Seeds of interest include grain seeds, such as corn, wheat, barley, rice, sorghum, rye, etc. Oil-seed plants include maize, palm, coconut, etc.

A "subject plant or plant cell" is one in which genetic alteration, such as transformation, has been effected as to a gene of interest, or is a plant or plant cell which is descended from a plant or cell so altered and which comprises the alteration. A "control" or "control plant" or "control plant cell" provides a reference point for measuring changes in phenotype of the subject plant or plant cell.

A control plant or plant cell may comprise, for example: (a) a wild-type plant or cell, i.e., of the same genotype as the starting material for the genetic alteration which resulted in the subject plant or cell; (b) a plant or plant cell of the same genotype as the starting material but which has been transformed with a null construct (i.e. with a construct which has no known effect on the trait of interest, such as a construct comprising a marker gene); (c) a plant or plant cell which is a non-transformed segregant among progeny of a subject plant or plant cell; (d) a plant or plant cell genetically identical to the subject plant or plant cell but which is not exposed to conditions or stimuli that would induce expression of the gene of interest; or (e) the subject plant or plant cell itself, under conditions in which the gene of interest is not expressed.

Any organism having a chimeric transcriptional control region comprising an enhancer domain of the invention or an active variant or fragment thereof are provided. In specific embodiments, plants, plant parts, cells, and germplasm having a chimeric transcriptional control region comprising an enhancer domain of the invention or an active variant or fragment thereof are provided. In specific embodiments, the chimeric transcriptional control region is operably linked to a polynucleotide of interest. Such polynucleotides include, for example, any polynucleotide that confers tolerance to a herbicide. Plant cells, plant parts and germplasm comprising such sequences are further provided.

### IV. Polynucleotides of Interest

The chimeric transcriptional regulatory control region having the enhancer domain or an active variant or fragment thereof can be used to express any polynucleotide of interest. General categories of polynucleotides of interest for the present invention include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, and environmental stress resistance (altered tolerance to cold, salt, drought, etc).

Insect resistance genes may encode resistance to pests that have great yield drag such as rootworm, cutworm, European corn borer, and the like. Such genes include, for example, *Bacillus thuringiensis* toxic protein genes (U.S. Patent Nos. 5,366,892; 5,747,450; 5,736,514; 5,723,756; 5,593,881; and Geiser et al. (1986) Gene 48:109); and the like.

Genes encoding disease resistance traits include detoxification genes, such as those which detoxify fumonisin (U.S. Patent No. 5,792,931); avirulence (avr) and disease resistance (R) genes (Jones et al. (1994) Science 266:789; Martin et al. (1993) Science 262:1432; and Mindrinos et al. (1994) Cell 78:1089); and the like.

Exogenous products include plant enzymes and products as well as those from other sources including prokaryotes and other eukaryotes. Such products include enzymes, cofactors, hormones, and the like.

Examples of other applicable genes and their associated phenotype include the gene which encodes viral coat protein and/or RNA, or other viral or plant genes that confer viral resistance; genes that confer fungal resistance; genes that promote yield improvement; and genes that provide for resistance to stress, such as cold, dehydration resulting from drought, heat and salinity, toxic metal or trace elements, or the like.

As noted, the polynucleotide operably linked to the transcription control region comprising the enhancer domain herein may be an antisense sequence for a targeted gene. Thus, the promoter sequences disclosed herein may be operably linked to antisense DNA sequences to reduce or inhibit expression of a native protein in the plant embryo.

"RNAi" refers to a series of related techniques to reduce the expression of genes (See for example U.S. Patent No. 6,506,559). Older techniques referred to by other names are now thought to rely on the same mechanism, but are given different names in the literature. These include "antisense inhibition," the production of antisense RNA transcripts capable of suppressing the expression of the target protein, and "co-suppression" or "sense-suppression," which refer to the production of sense RNA transcripts capable of suppressing the expression of identical or substantially similar foreign or endogenous genes (U.S. Patent No. 5,231,020). Such techniques rely on the use of constructs resulting in the accumulation of double stranded RNA with one strand complementary to the target gene to be silenced. The 35S enhancer in the context of a transcription control region of the embodiments may be used to drive expression of constructs that will result in RNA interference including microRNAs and siRNAs.

Where appropriate, the polynucleotides may be optimized for increased expression in the transformed plant. That is, the polynucleotides can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

In one embodiment, the polynucleotide of interest encodes a polynucleotide that confers tolerance to a herbicide of interest. In one embodiment, the polynucleotide that confers tolerance to herbicide of interest comprises an ALS inhibitor tolerant polypeptide which confers tolerance of a dose of sulfonylurea, imidazolinone, triazolopyrimidines, pyrimidinyoxy(thio)benzoates, and/or sulfonylamino-carbonyl-triazonline herbicide. Sulfonylurea and imidazolinone herbicides inhibit growth of higher plants by blocking acetolactate synthase (ALS), also known as, acetohydroxy acid synthase (AHAS). For example, plants containing particular mutations in ALS (*e.g.*, the S4 and/or HRA mutations) are tolerant to sulfonylurea herbicides. The production of sulfonylurea-tolerant plants and imidazolinone-tolerant plants is described more fully in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270. In specific embodiments, the ALS inhibitor tolerant polypeptide comprises a sulfonamide-tolerant acetolactate synthase, a sulfonamide-tolerant acetohydroxy acid synthase, an imidazolinone-tolerant acetolactate synthase, or an imidazolinone-tolerant acetohydroxy acid synthase.

Polynucleotides coding for resistance to herbicides that act to inhibit action of glutamine synthase, such as phosphinothricin or basta (e.g., the bar gene), glyphosate (e.g., the EPSPS gene and the GAT gene; see, for example, U.S. Publication No. 20040082770 and WO 03/092360) or other such genes known in the art can also be used. The *bar* gene encodes resistance to the herbicide basta, the *nptII* gene encodes resistance to the antibiotics kanamycin and geneticin, and the ALS gene mutants encode resistance to the herbicide chlorsulfuron.

Glyphosate resistance is imparted by mutant 5-enolpyruvl-3-phosphikimate synthase (EPSP) and *aroA* genes. See, for example, U.S. Patent No. 4,940,835 to Shah et al., which discloses the nucleotide sequence of a form of EPSPS which can confer glyphosate resistance. U.S. Patent No. 5,627,061 to Barry et al. also describes genes encoding EPSPS enzymes. See also U.S. Patent Nos. 6,248,876 B1; 6,040,497; 5,804,425; 5,633,435; 5,145;783; 4,971,908; 5,312,910; 5,188,642; 4,940,835; 5,866,775; 6,225,114 B1; 6,130,366; 5,310,667; 4,535,060; 4,769,061; 5,633,448; 5,510,471; Re. 36,449; RE 37,287 E; and 5,491,288; and international publications WO 97/04103; WO 97/04114; WO 00/66746; WO 01/66704; WO 00/66747 and WO 00/66748. Glyphosate resistance is also imparted to plants that express a gene that encodes a glyphosate oxido-reductase enzyme as described more fully in U.S. Patent Nos. 5,776,760 and 5,463,175. In addition glyphosate resistance can be imparted to plants by the over expression of genes encoding glyphosate N-acetyltransferase. See, for example, U.S. Patent Application Serial Nos. 10/004,357 and 10/427,692.

Polypeptides conferring tolerance to herbicides which inhibit the enzyme glutamine synthase, such as phosphinothricin or glufosinate (*e.g.*, the *bar* gene) can also be used. Glutamine synthetase (GS) appears to be an essential enzyme necessary for the development and life of most plant cells, and inhibitors of GS are toxic to plant cells. Glufosinate herbicides have been developed based on the toxic effect due to the inhibition of GS in plants. These herbicides are non-selective; that is, they inhibit growth of all the different species of plants present. The development of plants containing an exogenous phosphinothricin acetyltransferase is described in U.S. Patent Nos. 5,969,213; 5,489,520; 5,550,318; 5,874,265; 5,919,675; 5,561,236; 5,648,477; 5,646,024; 6,177,616; and 5,879,903. Mutated phosphinothricin acetyltransferase having this activity are also disclosed.

In still other embodiments, polypeptides conferring tolerance to herbicides which inhibit protox (protoporphyrinogen oxidase) can be used. Protox is necessary for the production of chlorophyll, which is necessary for all plant survival. The protox enzyme serves as the target for a variety of herbicidal compounds. These herbicides also inhibit growth of all the different species of plants present. The development of plants containing altered protox activity which are resistant to these herbicides are described in U.S. Patent Nos. 6,288,306; 6,282,837; and 5,767,373; and international publication WO 01/12825.

In still other embodiments, polypeptides involving other modes of herbicide resistance are employed. For example, hydroxyphenylpyruvatedioxygenases are enzymes that catalyze the reaction in which para-hydroxyphenylpyruvate (HPP) is transformed into homogentisate. Molecules which inhibit this enzyme and which bind to the enzyme in order to inhibit transformation of the HPP into homogentisate are useful as herbicides. Plants more resistant to certain herbicides are described in U.S. Patent Nos. 6,245,968; 6,268,549; and 6,069,115; and international publication WO 99/23886. Mutated hydroxyphenylpyruvatedioxygenase having this activity are also disclosed.

Additional herbicides, include but are not limited to, an acetyl Co-A carboxylase inhibitor such as quizalofop-P-ethyl, a synthetic auxin such as quinclorac, a protoporphyrinogen oxidase (PPO) inhibitor herbicide (such as sulfentrazone), a pigment synthesis inhibitor herbicide such as a hydroxyphenylpyruvate dioxygenase inhibitor (*e.g.*, mesotrione or sulcotrione), a phosphinothricin acetyltransferase or a phytoene desaturase inhibitor like diflufenican or pigment synthesis inhibitor.

### V. Methods of Introducing

The DNA construct comprising the enhancer domain of the present invention operably linked to a heterologous promoter can be used to transform any organism of interest. In specific embodiments, the organism comprises a plant or part thereof. In this manner, genetically modified plants, plant cells, plant tissue, seed, embryos, and the like can be obtained.

The methods of the invention involve introducing a polypeptide or polynucleotide into a plant. "Introducing" is intended to mean presenting to the plant the polynucleotide in such a manner that the sequence gains access to the interior of a cell of the plant. The methods of the invention do not depend on a particular method for introducing a sequence into a plant, only that the polynucleotide or polypeptides gains access to the interior of at least one cell of the plant. Methods for introducing polynucleotide or polypeptides into plants are known in the art including, but not limited to, stable transformation methods, transient transformation methods, and virus-mediated methods.

"Stable transformation" is intended to mean that the nucleotide construct introduced into a plant integrates into the genome of the plant and is capable of being inherited by the progeny thereof. "Transient transformation" is intended to mean that a polynucleotide is introduced into the plant and does not integrate into the genome of the plant or a polypeptide is introduced into a plant.

Transformation protocols as well as protocols for introducing polypeptides or polynucleotide sequences into plants may vary depending on the type of plant or plant cell, i.e., monocot or dicot, targeted for transformation. Suitable methods of introducing polypeptides and polynucleotides into plant cells include microinjection (Crossway et al. (1986) Biotechniques 4:320-334), electroporation (Riggs et al. (1986) Proc. Natl. Acad. Sci. USA 83:5602-5606, *Agrobacterium-mediated* transformation (U.S. Patent No. 5,563,055 and U.S. Patent No. 5,981,840), direct gene transfer (Paszkowski et al. (1984) EMBO J. 3:2717-2722); and ballistic particle acceleration (see, for example, U.S. Patent Nos. 4,945,050; U.S. Patent No. 5,879,918; U.S. Patent No. 5,886,244; and, 5,932,782; Tomes et al. (1995) in Plant Cell, Tissue, and Organ Culture:. Fundamental Methods, ed. Gamborg and Phillips (Springer-Verlag, Berlin); McCabe et al. (1988) Biotechnology 6:923-926); and Lec1 transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*).

In specific embodiments, the DNA construct of the invention can be provided to a plant using a variety of transient transformation methods. Such transient transformation methods include, the DNA construct comprising the enhancer domain can be transiently transformed into the plant using techniques known in the art. Such techniques include viral vector system and the precipitation of the polynucleotide in a manner that precludes subsequent release of the DNA. Thus, the transcription from the particle-bound DNA can occur, but the frequency with which its released to become integrated into the genome is greatly reduced. Such methods include the use particles coated with polyethylimine (PEI; Sigma #P3143).

In other embodiments, the polynucleotide of the invention may be introduced into plants by contacting plants with a virus or viral nucleic acids. Generally, such methods involve incorporating a nucleotide construct of the invention within a viral DNA or RNA molecule. Further, it is recognized that promoters of the invention also encompass promoters utilized for transcription by viral RNA polymerases. Methods for introducing polynucleotides into plants and expressing an operably linked sequence, involving viral DNA or RNA molecules, are known in the art. See, for example, U.S. Patent Nos. 5,889,191, 5,889,190, 5,866,785, 5,589,367, 5,316,931, and Porta et al. (1996) Molecular Biotechnology 5:209-221.

Methods are known in the art for the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853. Briefly, the polynucleotide of the invention can be contained in transfer cassette flanked by two non-recombinogenic recombination sites. The transfer cassette is introduced into a plant having stably incorporated into its genome a target site which is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide of the invention, for example, an expression cassette of the invention, stably incorporated into their genome.

In certain embodiments the polynucleotides of the present invention can be stacked with any combination of polynucleotide sequences of interest in order to create plants with a desired trait. A trait, as used herein, refers to the phenotype derived from a particular sequence or groups of sequences. For example, the polynucleotides of the present invention may be stacked with any other polynucleotides encoding polypeptides having pesticidal and/or insecticidal activity and/or herbicidal activity, such as other *Bacillus thuringiensis* toxic proteins (described in U.S. Patent Nos. 5,366,892; 5,747,450; 5,737,514; 5,723,756; 5,593,881; and Geiser et al. (1986) Gene 48:109), lectins (Van Damme *et al.* (1994) *Plant Mol. Biol.* 24:825, pentin (described in U.S. Patent No. 5,981,722), and the like. The combinations generated can also include multiple copies of any one of the polynucleotides of interest. The polynucleotides of the present invention can also be stacked with any other gene or combination of genes to produce plants with a variety of desired trait combinations including, but not limited to, traits desirable for animal feed such as high oil genes (e.g., U.S. Patent No. 6,232,529); balanced amino acids (e.g., hordothionins (U.S. Patent Nos. 5,990,389; 5,885,801; 5,885,802; and 5,703,409); barley high lysine (Williamson et al. (1987) Eur. J. Biochem. 165:99-106; and WO 98/20122) and high methionine proteins (Pedersen et al. (1986) J. Biol. Chem. 261:6279; Kirihara et al. (1988) Gene 71:359; and Musumura et al. (1989) Plant Mol. Biol. 12:123)); increased digestibility (e.g., modified storage proteins (U.S. Application Serial No. 10/053,410, filed November 7, 2001); and thioredoxins (U.S. Application Serial No. 10/005,429, filed December 3, 2001)).

The polynucleotides of the present invention can also be stacked with traits desirable for disease or herbicide resistance (e.g., fumonisin detoxification genes (U.S. Patent No. 5,792,931); avirulence and disease resistance genes (Jones et al. (1994) Science 266:789; Martin et al. (1993) Science 262:1432; Mindrinos et al. (1994) Cell 78:1089); acetolactate synthase (ALS) mutants that lead to herbicide resistance such as the S4 and/or Hra mutations; inhibitors of glutamine synthase such as phosphinothricin or basta (e.g., bar gene); and glyphosate resistance (EPSPS gene)); and traits desirable for processing or process products such as high oil (e.g., U.S. Patent No. 6,232,529); modified oils (e.g., fatty acid desaturase genes (U.S. Patent No. 5,952,544; WO 94/11516)); modified starches (e.g., ADPG pyrophosphorylases (AGPase), starch synthases (SS), starch branching enzymes (SBE), and starch debranching enzymes (SDBE)); and polymers or bioplastics (e.g., U.S. Patent No. 5.602,321; beta-ketothiolase, polyhydroxybutyrate synthase, and acetoacetyl-CoA reductase (Schubert et al. (1988) J. Bacteriol. 170:5837-5847) facilitate expression of polyhydroxyalkanoates (PHAs)); the disclosures of which are herein incorporated by reference. One could also combine the polynucleotides of the present invention with polynucleotides providing agronomic traits such as male sterility (e.g., see U.S. Patent No. 5.583,210), stalk strength, flowering time, or transformation technology traits such as cell cycle regulation or gene targeting (e.g., WO 99/61619, WO 00/17364, and WO 99/25821).

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system. See, for example, WO99/25821, WO99/25854, WO99/25840, WO99/25855, and WO99/25853.

### VI Methods of Use

A method for modulating the concentration and/or activity of any polynucleotide or polypeptide encoded thereby is provided. In general, concentration and/or activity is increased or decreased by at least 1%, 5%, 10%, 20%, 30%, 40%, 50%, 60%, 70%, 80%, or 90% relative to a native control plant, plant part, or cell which did not have the sequence of the invention introduced. Modulation in the present invention may occur during and/or subsequent to growth of the plant to the desired stage of development. The expression level of the polynucleotide or polypeptide of interest may be measured directly, for example, by assaying for the level of the polypeptide or polynucleotide in the organism, or indirectly, for example, by measuring the activity of the polypeptide or polypeptide in the organism.

In specific embodiments, the DNA construct comprising the enhancer domain is introduced into the plant cell. Subsequently, a plant cell having the introduced sequence of the invention is selected using methods known to those of skill in the art such as, but not limited to, Southern blot analysis, DNA sequencing, PCR analysis, or phenotypic analysis. In one method, a population of cells is provided and a DNA construct comprising a chimeric transcriptional regulatory region of the invention operably linked to the polynucleotide comprising a selectable marker is introduced into at least one cell of the population. The population of cells is then contacted with an effective concentration of an appropriate selection agent; and, the plant cell expressing the polynucleotide is selected. The plant cells having the DNA construct are thereby identified. A plant or plant part altered or modified by the foregoing embodiments is grown under plant forming conditions for a time sufficient to modulate the concentration and/or activity of the polynucleotide operably linked to the transcriptional control region comprising the enhancer domain. Plant forming conditions are well known in the art and are discussed briefly elsewhere herein.

In one embodiment, the enhancer of the invention is employed to modulate the expression of two polynucleotides of interest. In such methods, a DNA construct having the enhancer of the invention positioned between and operably linked to a first and a second promoter is introduced into a plant. In such methods, the enhancer allows for a modulation in expression of both the first and the second promoters from a divergent direction. Exemplary, but non-limiting, examples of such DNA constructs comprise in the 5' to 3' or 3' to 5' orientation: a first polynucleotide of interest operably linked to a first promoter, operably linked to at least one copy of an enhancer of the invention, operably linked to a second promoter, operably linked to a second polynucleotide of interest. In specific embodiments, the enhancer sequence is heterologous to the first and the second enhancer sequence.

In some embodiments, the chimeric transcriptional regulatory region comprising the enhancer domain can be used in DNA constructs which are operably linked to a selectable marker, such as a polynucleotide that can confer tolerance to a herbicide. For example, a DNA construct comprising a chimeric transcriptional regulatory region comprising an enhancer domain of the invention is operably linked to a polynucleotide that can confers tolerance to a herbicide which can function as a selectable marker, *e.g.*, in a plant, bacteria, actinomycete, yeast, algae or other fungi. For example, an organism that has been transformed with such a DNA construct can be selected based on its ability to grow in the presence of a herbicide that would not allow a control organism to grow. As used herein "an effective concentration" of a selective agent is a concentration of an agent that allows cells expressing the polynucleotide of interest to survive, but cells not expressing the polynucleotide sequence of interest will not survive in the presence of the agent at that concentration.

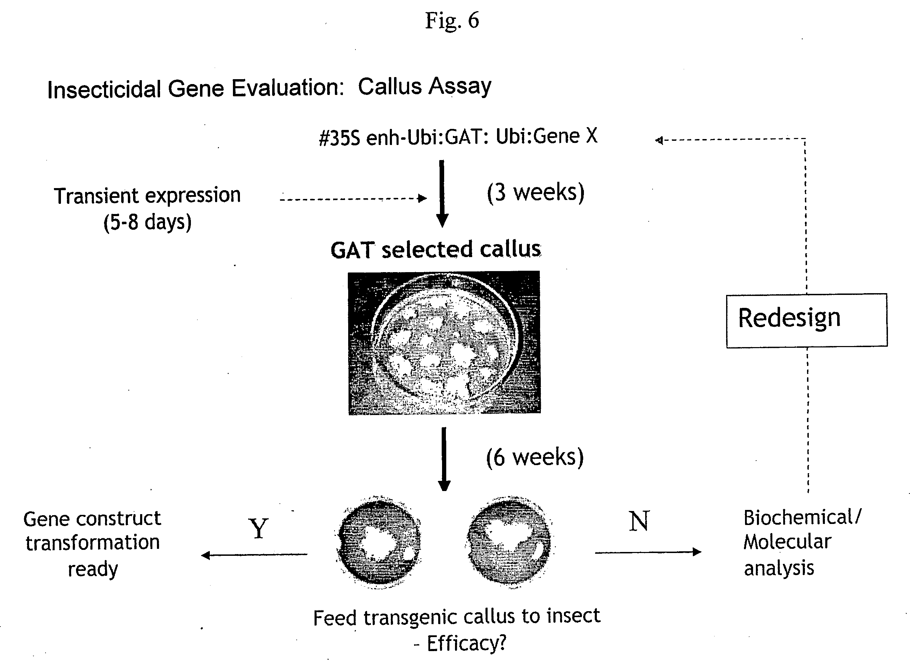

As demonstrated in Example 4 and Figure 6, such methods of selection allow one to evaluate expression of a polynucleotide of interest. For example, in specific embodiments such methods allow one to potential problems with the expression of a polynucleotide of interest at early stages in the transformation process. In such embodiments, the construct comprising the chimeric transcriptional regulatory region is operably linked to a polynucleotide encoding a selectable marker. The same construct further comprises a polynucleotide of interest which is also operably linked to the chimeric transcriptional regulatory region or to a separate promoter of interest. While any polynucleotide of interest can be employed, in specific examples, insecticidal polynucleotides are employed.

In other embodiments, a construct comprising a chimeric transcriptional regulatory region comprising an enhancer domain is operably linked to a polynucleotide of interest may exhibit a very high transformation efficiency, such as an efficiency of at least 20%, 30%, 40%, 50%, or 60% or higher. In specific embodiments, the polynucleotide of interest confers tolerance to a selectable marker, and in a more specific embodiment, the sequence confers tolerance to a herbicide of interest). In this manner, improved methods of transformation are provided. Moreover, when a construct comprising an enhancer domain of the invention is operably linked to a polynucleotide that can confer tolerance to a selectable marker (such as a sequence that confers herbicide tolerance), the transformants that are obtained may exhibit a very high frequency of tolerance to the marker, so that, for example, at least 20%, 30%, 40%, 50%, 60%, 70%, 80%, 90%, or 100% of the transformants are tolerant to the marker. As used herein, "transformation efficacy" is defined as the percentage of T0 events that display the desired phenotype of the polynucleotide of interest (i.e., display tolerance to a herbicide).

In addition, when a construct comprising a chimeric transcriptional regulatory domain comprising at least one copy of the enhancer domain is operably linked to a polynucleotide of interest, the frequency of transformation events in which only a single copy of the construct is inserted into the genome may be as high as at least 35%, 40%, 50%, 60%, 70%, 80%, 90%, or higher. In this manner, the invention also provides improved methods of transformation. It is recognized that multiple copies of the enhancer domain can be used, including 1, 2, 3, 4, 5, 6 or more. In such methods, the transformants may be selected using the appropriate method based on the polynucleotide introduced into the organism.

The invention further relates to a kit comprising at least one nucleic acid construct which comprises a chimeric transcriptional regulatory region comprising at least one copy of the enhancer domain of the invention or an active variant or fragment thereof. In some aspects, a construct of the invention will comprise a T-DNA sequence.

The following examples are offered by way of illustration and not by way of limitation.

### EXPERIMENTAL

### Example 1. Methods of Transformation Employing a GAT sequence in Maize

### I. Preparation ofAgrobacterium master plate

1. Obtain engineered *Agrobacterium tumefaciens* strain with GAT components (SEQ ID NO:7 or 8) and stored in -80°C degree freezer as a 50% glycerol stock. The transcriptional control region used was the 3X35S ENH (-) operably linked to the ZmUbi PRO-5UTR-ZmUbi intron 1 promoter (SEQ ID NO:9). This transcriptional control region (SEQ ID NO:9) is set forth below denoting the location of the various regions of the regulatory region: a) the 35S enhancer (3X) in the reverse direction has a single underline; b) the UBI promoter has a double underline, and c) the UBI intro is in italics.
2. Prepare master plate from a glycerol stock by streaking the bacteria to produce single colonies on #800 medium and incubate the bacteria at 28°C in the dark for 3-4 days.
3. Prepare a working plate by streaking 1 colony from the master plate across #810 media. Incubate bacteria at 28°C in the dark for 1-2 days.

### II. Preparation of bacteria for embryo infection

1. Prepare liquid culture of *Agrobacterium* 1 day prior to embryo isolation. Set up a flask with 30 mls of 557A medium, 30 µl of 2% acetosyringone and 30 µl of 5% spectinomycin.
2. Inoculate with 1 loopful of *Agrobacterium* from 810 medium and place on shaker (200 rpm) in dark room at 28°C overnight.
3. On morning of infection, take samples of the liquid culture of *Agrobacterium* and make a ¼ dilution with 557A. Use the diluted liquid culture to take OD reading using visible light at 550 nm.
4. Make dilutions to *Agrobacterium* culture as appropriate according the OD reading to maintain OD reading between 0.2-0.8 during embryo isolation.
5. When preparing *Agrobacterium* for infection, repeat OD reading of liquid culture. Using the OD reading calculate the number of mls required to obtain 5 E10 cfu/ml (cfu=colony forming unit) by using the formula EXPONENT (1.755 *(lnOD) + 21.77) as derived from a standard curve. Pipet the calculated amount of *Agrobacterium* liquid culture into 14 ml tube and centrifuge at 4500 rpm at 4 - 20 °C for ten minutes. Remove the supernatant and resuspend *Agrobacterium* in appropriate amount of 100 uM acetosyringone solution in 561Q.

### III. Immature embryo isolation

1. Harvest GS3 ears at 9-11 days after pollination with embryo size of 1-2 mm in length.
2. Sterilize ear in 50% bleach and 1 drop Tween for 20-30 minutes. Rinse 3-5 times in sterile water.
3. Isolate embryos from kernels and place in microtube containing 2 mls 561 Q.

### VI. Agrobacterium infection of embryos

1. Remove 561 Q with pipette from the microtube with isolated embryos and add 1 ml of Agrobacterium suspension at OD described above.
2. Mix by vortexing for about 30 seconds.
3. Allow 5 minutes for infection at room temperature.

### V. Co-cultivation

1. After removing liquid medium, transfer embryos and orient the embryos with embryonic axis down on the surface of 562P co-cultivation medium.
2. Place embryos in 20°C incubator for 3 days. Transfer to 28°C for 3 additional days.

### VI. Selection of transgenic putative callus events

1. After co-cultivation, transfer embryos to 563I selection medium containing 1 mM glyphosate. Culture the embryos at 28°C in dark.
2. Every 14-21 days transfer embryos to fresh 563I medium. The selection process may last about 2 months until actively growing putative callus events can be identified. Maintain putative callus events on 563I medium and sample callus for PCR.

### VII. Regeneration of T0 plants

1. Transfer callus events to 287I medium containing 0.1 mM Glyphosate until somatic embryos mature. Culture the callus at 28°C in dark.
2. Transfer mature embryos to 273I embryo germination medium containing 0.1 mM glyphosate in plates. Culture the plates at 28°C in light.
3. When shoots and roots emerge, transfer individual plants to 273I containing 0.1 mM Glyphosate in tubes. Culture the tubes at 28°C in light.
4. Plantlets with established shoots and roots shall be transferred to greenhouse for further growth and production of T1 seed.

### Example 2. Effect of 35S enhancer on Transformation Efficiency and Efficacy of GAT and ALS in Maize

### Materials and Methods

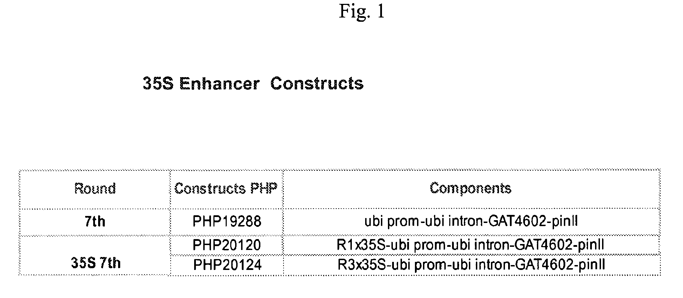

Two 35S enhancer constructs PHP20120, PHP20124) and one non-35S construct (PHP19288) were used to produce events to evaluate the effect of 35S enhancer on transformation efficiency and efficacy of GAT (SEQ ID NO:7) (Fig. 1). The differences between the two 35S enhancer constructs are the copy numbers of the 35S enhancer.

A summary of each 35S enhancer construct is provided below.

**PHP20120** comprises 35S ENH (-):ZmUBI PRO-5UTR-UBI INTRON1 (-denotes reverse direction of 35S enhancer). This transcriptional control region (SEQ ID NO:13) is set forth below denoting the location of the various regions of the regulatory region: a) the 35S enhancer in the reverse direction has a single underline; b) the UBI promoter has a double underline, and c) the UBI intron is in italics.

**PHP20124** comprises 3X35S ENH (-): ZmUBI PRO-5UTR-UBI INTRON1 (-denotes reverse direction of 35S enhancer). This transcriptional control region (SEQ ID NO:14) is set forth below denoting the location of the various regions of the
regulatory region: a) the 35S enhancer in the reverse direction has a single underline; b) the UBI promoter has a double underline, and c) the UBI intron is in italics.

The transformation experiments were conducted side-by-side using the same embryos from the same ears. Immature embryos of GS3 line were aseptically removed from each ear and divided into five portions. Each portion of the embryos was then infected with *A. tumefaciens* strain LBA4404 containing the expression cassettes from each of the five constructs, respectively. After 6 days co-cultivation, the embryos were transferred to fresh selection medium containing glyphosate. The transformed cells, which survived the glyphosate selection proliferated and produced somatic embryogenic calli. After about two months subculture, the calli were then manipulated to regenerate whole transgenic plants with glyphosate presence and were transferred to the greenhouse. T0 plants were then subjected to glyphosate spray at 6X (156 oz/ac) Roundup Ready UltraMax™ at V3 or V4 stage in the greenhouse. Positive plants were sampled for quantitative PCR for copy number and western for expression. T0 plants were then crossed with inbred lines to obtain seeds for further evaluation.

### Results

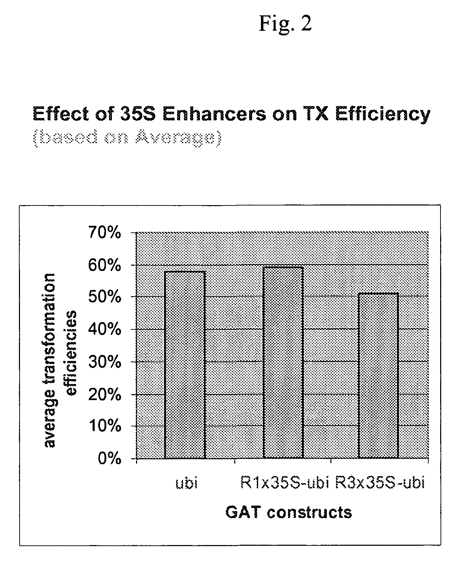

Transformation efficiency was measured as the percentage of the infected embryos that produced resistant calli after selection. The average transformation efficiencies for PHP19288, PHP20120, and PHP20124 were 58%, 59%, and 51%, respectively. The data indicated that all constructs had quite high and similar transformation efficiencies. (Fig 2).

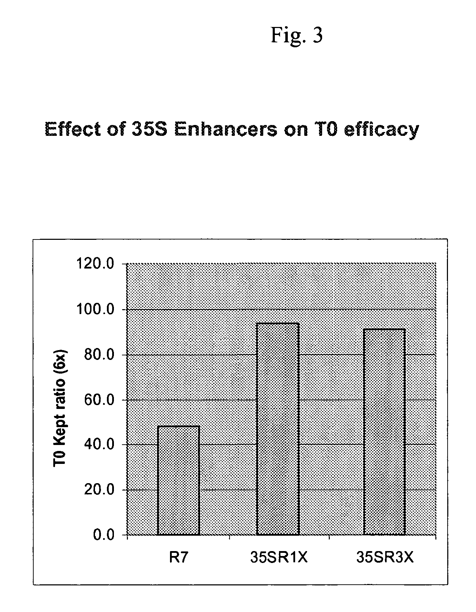

T0 plant efficacy was defined as the percentage of the T0 events that were completely resistant to the 6x glyphosate spray. The efficacy of the non-35S construct (PHP19288) was 48.1%. In contrast, the efficacies of the 35S enhancer constructs (PHP20120, and PHP20124) were 93.5%, and 91.1%, respectively (Fig. 3). The data showed that the 35S enhancer constructs significantly increased the plant efficacy against glyphosate.

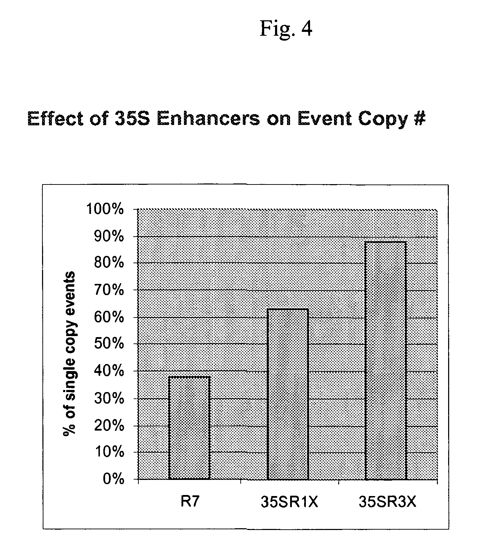

Another significant improvement of using 35S enhancer was in integration pattern of the transgene. The percentage of the tested events that were single copy for the non-35S enhancer construct was only 38%, but for the 35S enhancer constructs (PHP20120 and PHP20124) single copy events represented 63%, and 88% of the events, respectively (Fig. 4).

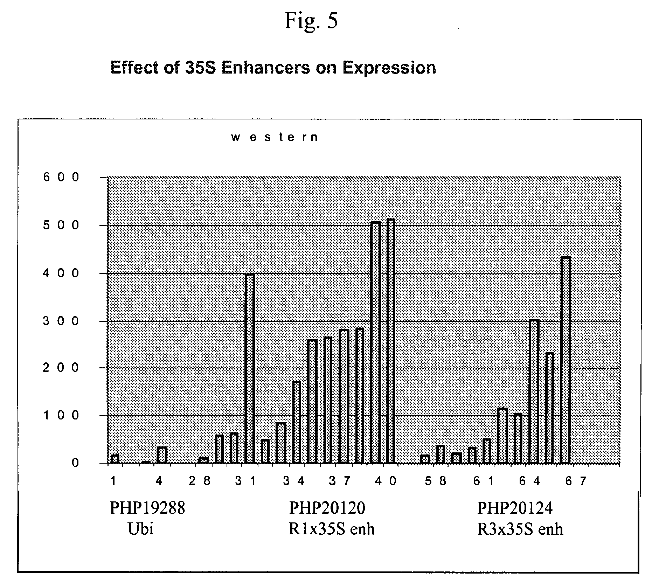

A subset of events from all three constructs were sampled by Western analysis to look any comparative differences in GAT expression between non 35S and 35S events. This analysis showed that events from the non-35S enhancer construct had very low levels of GAT expression whereas the majority of the events from the 35S enhancer constructs showed very high levels of GAT expression (Fig. 5).

### Example 3. Using 35S Enhancer GAT in Developing A Novel Callus-Based Gene/Construct Evaluation System.

### Materials and Methods

This assay is being developed to improve the evaluation of expression of an insecticidal gene at a very early stage in the transformation process in order to identify potential problems with expression. The basis of this assay is the use of the glyphosate acetyl transferase (GAT) gene (SEQ ID NO:8) as a selectable marker. Both GAT and the insecticidal test gene will be driven by a strong constitutive promoter and linked in the same construct. The promoter employed comprised the ZmUBI PRO-5UTR-UBI INTRON1 with the 3X35S enhancer as described above in Example 1. As a result it is expected that selection on high levels of glyphosate will identify high insecticidal test gene expressors. The callus tissue from these putative high expressors will then be used in insect bioassays to determine whether the gene product is functional. Those constructs showing efficacy can be advanced into transformation. If the construct does not show efficacy then follow up biochemical and molecular analyses can be conducted to identify the problem and the gene will be redesigned and retested in the system (Fig. 6).

### Results

Preliminary data has shown that the activity of an efficacious insect control gene can be detected at the callus stage. The correlation between the callus activity and the plant efficacy is currently being evaluated.

### Example 4. GAT as a Selectable Marker

### Materials and Method

*Agrobacterium* mediated transformation was used to introduce the *GAT* (SEQ ID NO:8) expression cassette into the corn genome. The *GAT* expression cassette comprises the promoter comprising ZmUBI PRO-5UTR-UBI INTRON1 with the 3X35S enhancer (as described above in Example 1) operably linked to the *gat* gene, and pinII terminator. *Agrobacterium tumefaciens,* strain LBA4404, was pathogenically disarmed by removing its native T-DNA. Instead, the T-DNA site on the Ti plasmid contained the *GAT* expression cassette.

Immature embryos of maize were aseptically removed from the developing caryopsis and treated with *A. tumefaciens* strain LBA4404 containing GAT expression cassettes. After a period of embryo and *Agrobacterium* co-cultivation on solid culture medium without glyphosate presence, the embryos were transferred to fresh selection medium that contained antibiotics and glyphosate. The antibiotics kill any remaining *Agrobacterium.* The selection medium is stimulatory to maize somatic embryogenesis and selective for those cells that contain an integrated *gat* gene. Therefore, callus that survives glyphosate to proliferate and produce embryogenic tissue is presumably genetically transformed. Callus samples were taken for molecular analysis to verify the presence of the transgene by PCR. The embryonic tissue is then manipulated to regenerate transgenic plants in the presence of glyphosate that are then transferred to the greenhouse. T0 plants are sprayed with glyphosate at different concentrations. Positive plants are sampled for molecular analysis for transgene copy number and crossed with inbred lines to obtain seeds from the initially transformed plants.

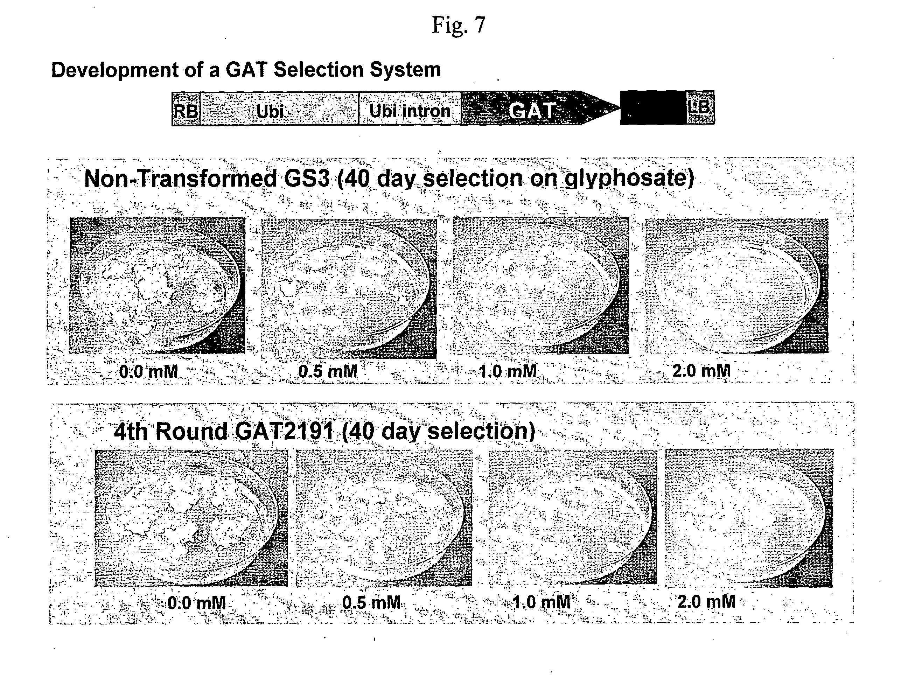

A glyphosate kill curve was established by testing non-transformed embryos response on media with different levels of glyphosate. GS3 embryos were isolated from an immature ear and placed onto media containing glyphosate at 0,0, 0.5, 1.0, and 2.0 mM. After about 40 days culture, the response of the embryos were observed and recorded. Similarly, infected GS3 embryos with the GAT construct were placed onto media containing glyphosate at 0.0, 0.5*,* 1.0, and 2.0 mM. After about 40 days culture, the response of the infected embryos were observed and recorded (Fig. 7).

A side-by-side experiment was conducted to compare the transformation efficiencies of GAT, bar and mopat Immature embryos of GS3 line were aseptically removed from each ear and divided into three portions. Each portion of the embryos was then infected with *A. tumefaciens* strain LBA4404 containing the expression cassettes of GAT, bar, or mopat respectively. After co-cultivation, the embryos infected with GAT construct were selected on routine glyphosate medium and the embryos infected with bar or mopat constructs were selected on routine glufosinate medium. The subcultures were done every 2 weeks. At about 50 days selection the responses of the embryos were observed and recorded.

### Results

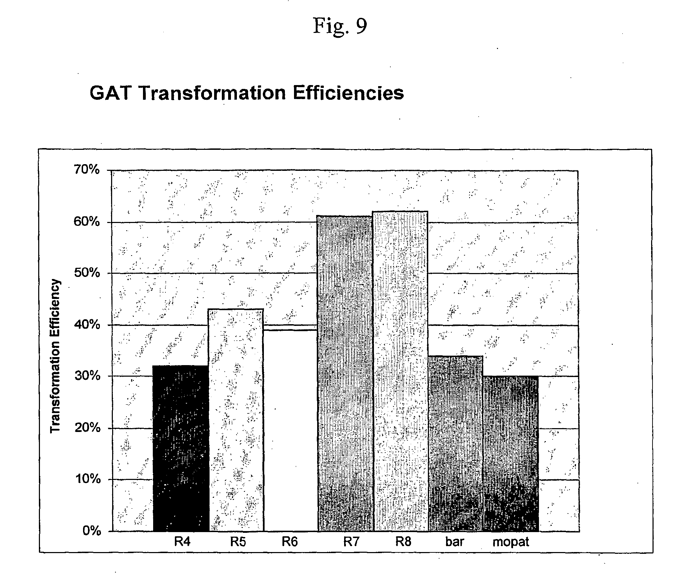

From the glyphosate kill curve experiment, all embryos on medium with 0.0 mM glyphosate initiated healthy callus, while about half of the embryos on medium with 0.5 mM glyphosate showed callus initiation. There was very little callus growth with embryos on media containing 1.0 and 2.0 mm glyphosate. This indicated that 0.5 mM is not enough to inhibit all embryos growth, but 1 mM or 2 mM is strong enough to kill the non-transformed embryos. In the infected embryo experiment, more callus was grown on media with 0.0 and 0.5 mM glyphosate, but some embryos initiated resistant callus on media with 1.0 mM or 2.0 mM glyphosate. Western or PCR analysis has confirmed that these resistant calli were transformed. Currently, GAT has performed consistently as an effective selectable marker with excellent transformation efficiency in both GS3 and introEF09B genotypes (Fig. 9 and Table 1).

**Table 1 GAT transformation efficiency in introEF09B**

| genotype | construct | selectable marker | # infected embryos | # events to GH | txn % based on # events to GH |
|---|---|---|---|---|---|
| EFWWBTX | GATHRA | GAT | 1332 | 354 | 27% |
| EFWWCTX | GATHRA | GAT | 136 | 47 | 35% |
| EFWWETX | GATHRA | GAT | 1109 | 158 | 14% |
| EFWWZTX | GATHRA | GAT | 1790 | 502 | 28% |
| | | | 4367 | 1061 | 24% |

In the side-by-side experiment to compare GAT, bar and mopat, GAT gave the best transformation efficiency at about 64%, bar at 34%, and mopat at 30%. Calli with GAT selection seem to grow faster that those selected on glufosinate (Fig. 9).

### Example 5: Transformation and Degeneration of Transgenic Plants (for reference only)

Immature maize embryos from greenhouse donor plants are bombarded with a plasmid containing a chimeric transcriptional regulatory region comprising the enhancer domain of SEQ ID NO:1 operably linked to ZmUBI PRO-5UTR-ZmUBI INTRON1 (SEQ ID NO:6). The chimeric transcriptional regulatory region is operably linked to a polynucleotide of interest and the selectable marker gene PAT (Wohlleben et al. (1988) Gene 70:25-37), which confers resistance to the herbicide Bialaphos. Alternatively, the selectable marker gene is provided on a separate plasmid. Transformation is performed as follows. Media recipes follow below.

### Preparation of Target Tissue

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5cm target zone in preparation for bombardment.

A plasmid vector comprising the construct described above is made. This plasmid DNA plus plasmid DNA containing a PAT selectable marker is precipitated onto 1.1 µm (average diameter) tungsten pellets using a CaCl₂ precipitation procedure as follows: 100 µl prepared tungsten particles in water; 10 µl (1 µg) DNA in Tris EDTA buffer (1 µg total DNA); 100 µl 2.5 M CaCl₂; and 10 µl 0.1 M spermidine.

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment

The sample plates are bombarded at level #4 in a particle gun. All samples receive a single shot at 650 PSI, with a total of ten aliquots taken from each tube of prepared particles/DNA.

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity. Plants are monitored and scored for the desired phenotype based on the polynucleotide of interest.

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H₂O following adjustment to pH 5.8 with KOH); 2.0 g/l Gelrite (added after bringing to volume with D-I H₂O); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/12,4-D (brought to volume with D-I H₂O following adjustment to pH 5.8 with KOH); 3.0 g/l Gelrite (added after bringing to volume with D-I H₂O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H₂O) (Murashige and Skoog (1962) Physical. Plant. 15:473), 100 mg/l myo-inositol, 0.5 mg/l zeatin, 60 g/l sucrose, and 1.0 ml/l of 0.1 mM abscisic acid (brought to volume with polished D-I H₂O after adjusting to pH 5.6); 3.0 g/l Gelrite (added after bringing to volume with D-I H₂O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H₂O), 0.1 g/l myo-inositol, and 40.0 g/l sucrose (brought to volume with polished D-I H₂O after adjusting pH to 5.6); and 6 g/l bacto-agar (added after bringing to volume with polished D-I H₂O), sterilized and cooled to 60°C.

### Example 6. Soybean Embryo Transformation (for reference only)

### Culture Conditions

Soybean embryogenic suspension cultures (cv. Jack) are maintained in 35 ml liquid medium SB196 (see recipes below) on rotary shaker, 150 rpm, 26°C with cool white fluorescent lights on 16:8 hr day/night photoperiod at light intensity of 60-85 µE/m2/s. Cultures are subcultured every 7 days to two weeks by inoculating approximately 35 mg of tissue into 35 ml of fresh liquid SB196 (the preferred subculture interval is every 7 days).

Soybean embryogenic suspension cultures are transformed with the plasmids and DNA fragments described in the following examples by the method of particle gun bombardment (Klein et al. (1987) Nature, 327:70).

### Soybean Embryogenic Suspension Culture Initiation

Soybean cultures are initiated twice each month with 5-7 days between each initiation.

Pods with immature seeds from available soybean plants 45-55 days after planting are picked, removed from their shells and placed into a sterilized magenta box. The soybean seeds are sterilized by shaking them for 15 minutes in a 5% Clorox solution with 1 drop of ivory soap (95 ml of autoclaved distilled water plus 5 ml Clorox and 1 drop of soap). Mix well. Seeds are rinsed using 2 1-liter bottles of sterile distilled water and those less than 4 mm are placed on individual microscope slides. The small end of the seed are cut and the cotyledons pressed out of the seed coat. Cotyledons are transferred to plates containing SB1 medium (25-30 cotyledons per plate). Plates are wrapped with fiber tape and stored for 8 weeks. After this time secondary embryos are cut and placed into SB 196 liquid media for 7 days.

### Preparation of DNA for Bombardment

Either an intact plasmid or a DNA plasmid fragment containing the genes of interest and the selectable marker gene are used for bombardment. Plasmid DNA for bombardment are routinely prepared and purified using the method described in the Promega™ Protocols and Applications Guide, Second Edition (page 106). Fragments of the plasmids carrying the chimeric transcriptional regulatory region comprising the enhancer domain of SEQ ID NO:1 operably linked to ZmUBI PRO-5UTR-ZmUBI INTRON1 (SEQ ID NO:6) which is operably linked to a polynucleotide of interest are obtained by gel isolation of double digested plasmids. In each case, 100 µg of plasmid DNA is digested in 0.5 ml of the specific enzyme mix that is appropriate for the plasmid of interest. The resulting DNA fragments are separated by gel electrophoresis on 1% SeaPlaque GTG agarose (BioWhitaker Molecular Applications) and the DNA fragments containing construct described above are cut from the agarose gel. DNA is purified from the agarose using the GELase digesting enzyme following the manufacturer's protocol.

A 50 µl aliquot of sterile distilled water containing 3 mg of gold particles (3 mg gold) is added to 5 µl of a 1 µg/µl DNA solution (either intact plasmid or DNA fragment prepared as described above), 50 µl 2.5M CaCl₂ and 20 µl of 0.1 M spermidine. The mixture is shaken 3 min on level 3 of a vortex shaker and spun for 10 sec in a bench microfuge. After a wash with 400 µl 100% ethanol the pellet is suspended by sonication in 40 µl of 100% ethanol. Five µl of DNA suspension is dispensed to each flying disk of the Biolistic PDS 1000/HE instrument disk. Each 5 µl aliquot contains approximately 0.375 mg gold per bombardment (i.e. per disk).

### Tissue Preparation and Bombardment with DNA

Approximately 150-200 mg of 7 day old embryonic suspension cultures are placed in an empty, sterile 60 x 15 mm petri dish and the dish covered with plastic mesh. Tissue is bombarded 1 or 2 shots per plate with membrane rupture pressure set at 1100 PSI and the chamber evacuated to a vacuum of 27-28 inches of mercury. Tissue is placed approximately 3.5 inches from the retaining / stopping screen.

### Selection of Transformed Embryos

Transformed embryos were selected either using hygromycin (when the hygromycin phosphotransferase, HPT, gene was used as the selectable marker) or chlorsulfuron (when the acetolactate synthase, ALS, gene was used as the selectable marker).

### Hygromycin (HPT) Selection

Following bombardment, the tissue is placed into fresh SB196 media and cultured as described above. Six days post-bombardment, the SB 196 is exchanged with fresh SB196 containing a selection agent of 30 mg/L hygromycin. The selection media is refreshed weekly. Four to six weeks post selection, green, transformed tissue may be observed growing from untransformed, necrotic embryogenic clusters. Isolated, green tissue is removed and inoculated into multiwell plates to generate new, clonally propagated, transformed embryogenic suspension cultures.

### Chlorsulfuron (ALS) Selection

Following bombardment, the tissue is divided between 2 flasks with fresh SB196 media and cultured as described above. Six to seven days post-bombardment, the SB 196 is exchanged with fresh SB 196 containing selection agent of 104 ng/ml Chlorsulfuron. The selection media is refreshed weekly. Four to six weeks post selection, green, transformed tissue may be observed growing from untransformed, necrotic embryogenic clusters. Isolated, green tissue is removed and inoculated into multiwell plates containing SB196 to generate new, clonally propagated, transformed embryogenic suspension cultures.

### Regeneration of Soybean Somatic Embryos into Plants

In order to obtain whole plants from embryogenic suspension cultures, the tissue must be regenerated.

### Embryo Maturation

Embryos are cultured for 4-6 weeks at 26°C in SB 196 under cool white fluorescent (Phillips cool white Econowatt F40/CW/RS/EW) and Agro (Phillips F40 Agro) bulbs (40 watt) on a 16:8 hr photoperiod with light intensity of 90-120 uE/m2s, After this time embryo clusters are removed to a solid agar media, SB 166, for 1-2 weeks. Clusters are then subcultured to medium SB103 for 3 weeks. During this period, individual embryos can be removed from the clusters and screened for the desired phenotype based on the polynucleotide of interest employed. It should be noted that any detectable phenotype, resulting from the expression of the genes of interest, could be screened at this stage.

### Embryo Desiccation and Germination

Matured individual embryos are desiccated by placing them into an empty, small petri dish (35 x 10 mm) for approximately 4-7 days. The plates are sealed with fiber tape (creating a small humidity chamber). Desiccated embryos are planted into SB71-4 medium where they were left to germinate under the same culture conditions described above. Germinated plantlets are removed from germination medium and rinsed thoroughly with water and then planted in Redi-Earth in 24-cell pack tray, covered with clear plastic dome. After 2 weeks the dome is removed and plants hardened off for a further week. If plantlets looked hardy they are transplanted to 10" pot of Redi-Earth with up to 3 plantlets per pot. After 10 to 16 weeks, mature seeds are harvested, chipped and analyzed for proteins.

### Media Recipes

| | |
|---|---|
| SB 196 - FN Lite liquid proliferation medium (per liter) - MS FeEDTA - 100x Stock 1 | 10 ml |
| MS Sulfate - 100x Stock 2 | 10 ml |
| FN Lite Halides - 100X Stock 3 | 10 ml |
| FN Lite P,B,Mo - 100x Stock 4 | 10 ml |
| B5 vitamins (1ml/L) | 1.0 ml |
| 2,4-D (10mg/L final concentration) | 1.0 ml |
| KNO3 | 2.83 gm |
| (NH4)2 SO 4 | 0.463 gm |
| Asparagine | 1.0 gm |
| Sucrose (1%) | 10 gm |
| pH 5.8 | |

### FN Lite Stock Solutions

| Stock # | | | 1000ml | 500ml |
|---|---|---|---|---|
| 1 | MS Fe EDTA | 100x Stock | | |
| | | Na₂ EDTA* | 3.724 g | 1.862 g |
| | | FeSO₄-7H₂O | 2.784 g | 1.392 g |
| | | * Add first, dissolve in dark bottle while stirring | | |
| 2 | | MS Sulfate 100x stock | | |
| | | MgSO₄-7H₂O | 37.0 g | 18.5 g |
| | | MnSO₄-H₂O | 1.69 g | 0.845 g |
| | | ZnSO₄-7H₂O | 0.86 g | 0.43 g |
| | | CuSO₄ - 5H₂O | 0.0025 g | 0.00125 g |
| 3 | FN Lite Halides | 100x Stock | | |
| | | CaCl₂-2H₂O | 30.0 g | 15.0 g |
| | | KI | 0.083 g | 0.0715 g |
| | | CoCl₂ - 6H₂O | 0.0025 g | 0.00125 g |
| 4 | | FN Lite P,B,Mo 100x Stock | | |
| | | KH₂PO₄ | 18.5 g | 9.25 g |
| | | H₃BO₃ | 0.62 g | 0.31 g |
| | | Na₂MoO₄ - 2H₂O | 0.025 g | 0.0125 g |

SB 1 solid medium (per liter) comprises: 1 pkg. MS salts (Gibco/ BRL - Cat# 11117-066); 1 ml B5 vitamins 1000X stock; 31.5 g sucrose; 2 ml 2,4-D (20mg/L final concentration); pH 5.7; and, 8 g TC agar.

SB 166 solid medium (per liter) comprises: 1 pkg. MS salts (Gibco/ BRL - Cat# 11117-066); 1 ml B5 vitamins 1000X stock; 60 g maltose; 750 mg MgCl2 hexahydrate; 5 g activated charcoal; pH 5.7; and, 2 g gelrite.

SB 103 solid medium (per liter) comprises: 1 pkg. MS salts (Gibco/BRL - Cat# 11117-066); 1 ml B5 vitamins 1000X stock; 60 g maltose; 750 mg MgCl2 hexahydrate; pH 5.7; and, 2 g gelrite.

SB 71-4 solid medium (per liter) comprises: 1 bottle Gamborg's B5 salts w/ sucrose (Gibco/BRL - Cat# 21153-036); pH 5.7; and, 5 g TC agar.

2,4-D stock is obtained premade from Phytotech cat# D 295 - concentration is I mg/ml.

B5 Vitamins Stock (per 100 ml) which is stored in aliquots at -20C comprises: 10 g myo-inositol; 100 mg nicotinic acid; 100 mg pyridoxine HCl; and, 1 g thiamine. If the solution does not dissolve quickly enough, apply a low level of heat via the hot stir plate. Chlorsulfuron Stock comprises 1mg / ml in 0.01 N Ammonium Hydroxide

The article "a" and "an" are used herein to refer to one or more than one (i.e., to at least one) of the grammatical object of the article. By way of example, "an element" means one or more element.

All publications and patent applications mentioned in the specification are indicative of the level of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. A method of expressing a polynucleotide of interest comprising
introducing into a monocot plant cell at least one DNA construct comprising a chimeric transcriptional regulatory region comprising at least one enhancer domain operably linked to a heterologous promoter, said chimeric transcriptional regulatory region operably linked to the polynucleotide of interest, wherein said enhancer domain is selected from the group consisting of:
(a) the nucleotide sequence comprising SEQ ID NO: 17 or at least 3 copies of SEQ ID NO:1, wherein said enhancer domain is in the reverse orientation and operably linked to said promoter;
(b) the nucleotide sequence comprising at least 95% sequence identity to SEQ ID NO: 17, wherein said nucleotide sequence has transcriptional regulatory activity and wherein said enhancer domain is in the reverse orientation and operably linked to said promoter; or,
(c) a nucleotide sequence comprising at least 3 copies of a sequence having 95% sequence identity to SEQ ID NO: 1, wherein said nucleotide sequence retains transcriptional regulatory activity, wherein said enhancer domains are in the reverse orientation and operably linked to said promoter.

2. The method of claim 1, wherein said enhancer domain does not comprise the sequence set forth in SEQ ID NO: 5.

3. The method of claim 1, wherein said copies of said enhancer are immediately adjacent to one another.

4. The method of claim 1, 2, or 3, wherein said polynucleotide of interest encodes
(a) a polypeptide; and/or
(b) a selectable marker.

5. The method of claim 4, wherein said selectable marker comprises a polynucleotide that confers tolerance to a herbicide.

6. The method of claim 5, wherein said method further comprises culturing said monocot plant cell in a media comprising an effective concentration of the herbicide.

7. A method to select a monocot plant cell having a polynucleotide of interest comprising
a) providing a population of monocot plant cells;
b) introducing into at least one of the monocot plant cells from said population a DNA construct comprising the polynucleotide of interest and further comprising a chimeric transcriptional regulatory region comprising at least one enhancer domain operably linked to a first heterologous promoter, said chimeric transcriptional regulatory region operably linked to a selectable marker, wherein said enhancer domain is selected from the group consisting of:
i) the nucleotide sequence comprising SEQ ID NO: 17 or at least 3 copies of SEQ ID NO:1, and wherein said enhancer domain is in the reverse orientation and operably linked to said promoter;
ii) the nucleotide sequence comprising at least 95% sequence identity to SEQ ID NO:17, wherein said nucleotide sequence has transcriptional regulatory activity, wherein said enhancer domain is in the reverse orientation and operably linked to said promoter; and,
iii) a nucleotide sequence comprises at least 3 copies of a sequence having 95% sequence identity to SEQ ID NO: 1, wherein said nucleotide sequence retains transcriptional regulatory activity, and wherein said enhancer domains are in the reverse orientation and operably linked to said promoter;
c) contacting said population of the monocot plant cells with an effective concentration of an appropriate selection agent; and,
d) selecting the monocot plant cell expressing said selectable marker, and thereby identifying a monocot plant cell having the polynucleotide of interest.

8. The method of claim 7, wherein said enhancer domain does not comprise the sequence set forth in SEQ ID NO: 5.

9. The method of claim 7, wherein the copies of said enhancer are immediately adjacent to one another.

10. The method of claim 7, 8, or 9, wherein said selectable marker encodes a polypeptide that confers tolerance to a herbicide.

11. The method of claim 10, wherein said selective agent comprises a herbicide.

12. The method of claim 7, wherein said DNA construct comprises in the 5' to 3' or 3' to 5' orientation: the first polynucleotide of interest operably linked to a second heterologous promoter, operably linked to at least one of said enhancer domains, operably linked to the first heterologous promoter, operably linked to the selectable marker, wherein said first polynucleotide of interest and said selectable marker are expressed in divergent directions.

13. A polynucleotide comprising a chimeric transcriptional regulatory element comprising a promoter that drives expression in a cell operably linked to at least one copy of a heterologous enhancer domain wherein said enhancer domain is selected from the group consisting of:
(a) the nucleotide sequence comprising SEQ ID NO:17 or at least 3 copies of SEQ ID NO:1 wherein said enhancer domain is in the reverse orientation and operably linked to said promoter;
(b) the nucleotide sequence comprising at least 95% sequence identity to SEQ ID NO: 17, wherein said nucleotide sequence has regulating transcriptional activity, and is in the reverse orientation and operably linked to said promoter; or,
(c) a nucleotide sequence comprises at least 3 copies of a sequence having 95% sequence identity to SEQ ID NO: 1, wherein said nucleotide sequence retains transcriptional regulatory activity, wherein said enhancer domains are in the reverse orientation and operably linked to said promoter.

14. The polynucleotide of claim 13, wherein said enhancer domain does not comprise the sequence of SEQ ID NO: 5.

15. The polynucleotide of claim 13, wherein the copies of said enhancer are immediately adjacent to one another.

16. An expression vector, monocot plant cell or monocot plant comprising the polynucleotide of any one of claims 13 to 15.

17. The method of any one of claims 1 to 6 or 7 to 12, wherein said monocot is maize, wheat, rice, barley, sorghum, or rye.

18. The method of any one of claims 1-12 or 17 wherein said method increases a single copy integration event in the monocot plant cell.

## Patentansprüche

1. Verfahren zum Exprimieren eines Polynucleotids, das von Interesse ist, umfassend
das Einführen in eine Zelle einer einkeimblättrigen Pflanze mindestens eines DNA-Konstrukts umfassend eine chimäre transkriptionelle regulatorische Region umfassend mindestens eine Enhancerdomäne, die funktionsfähig mit einem heterologen Promotor verknüpft ist, wobei die chimäre transkriptionelle regulatorische Region mit dem Polynucleotid, das von Interesse ist, funktionsfähig verknüpft ist, wobei die Enhancerdomäne aus der Gruppe ausgewählt ist bestehend aus:
(a) der Nucleotidsequenz, die SEQ ID NO: 17 oder mindestens 3 Kopien von SEQ ID NO. 1 umfasst, wobei die Enhancerdomäne sich in umgekehrter Orientierung befindet und funktionsfähig mit dem Promotor verknüpft ist;
(b) der Nucleotidsequenz, die mindestens 95 % Sequenzidentität mit SEQ ID NO: 17 umfasst, wobei die Nucleotidsequenz eine transkriptionelle regulatorische Aktivität aufweist und wobei die Enhancerdomäne sich in umgekehrter Orientierung befindet und funktionsfähig mit dem Promotor verknüpft ist; oder
(c) einer Nucleotidsequenz, die mindestens 3 Kopien einer Sequenz umfasst, die 95 % Sequenzidentität mit SEQ ID NO: 1 aufweist, wobei die Nucleotidsequenz eine transkriptionelle regulatorische Aktivität beibehält, wobei die Enhancerdomänen sich in umgekehrter Orientierung befinden und funktionsfähig mit dem Promotor verknüpft sind.

2. Verfahren nach Anspruch 1, wobei die Enhancerdomäne die in SEQ ID NO: 5 aufgeführte Sequenz nicht umfasst.

3. Verfahren nach Anspruch 1, wobei die Kopien des Enhancers direkt aneinander anliegen.

4. Verfahren nach Anspruch 1, 2 oder 3, wobei das Polynucleotid, das von Interesse ist, für
(a) ein Polypeptid; und/oder
(b) einen selektierbaren Marker kodiert.

5. Verfahren nach Anspruch 4, wobei der selektierbare Marker ein Polynucleotid umfasst, das Toleranz für ein Herbizid verleiht.

6. Verfahren nach Anspruch 5, wobei das Verfahren des Weiteren das Züchten der Zelle der einkeimblättrigen Pflanze in einem Medium umfasst, das eine wirksame Konzentration des Herbizids umfasst.

7. Verfahren zum Selektieren einer Zelle einer einkeimblättrigen Pflanze, die ein Polynucleotid aufweist, das von Interesse ist, umfassend
a) das Bereitstellen einer Population von Zellen einer einkeimblättrigen Pflanze;
b) das Einführen in mindestens eine der Zellen der einkeimblättrigen Pflanze aus der Population eines DNA-Konstrukts umfassend das Polynucleotid, das von Interesse ist, und des Weiteren umfassend eine chimäre transkriptionelle regulatorische Region umfassend mindestens eine Enhancerdomäne, die funktionsfähig mit einem ersten heterologen Promotor verknüpft ist, wobei die chimäre transkriptionelle regulatorische Region mit einem selektierbaren Marker funktionsfähig verknüpft ist, wobei die Enhancerdomäne aus der Gruppe ausgewählt ist bestehend aus:
i) der Nucleotidsequenz, die SEQ ID NO: 17 oder mindestens 3 Kopien von SEQ ID NO. 1 umfasst, wobei die Enhancerdomäne sich in umgekehrter Orientierung befindet und funktionsfähig mit dem Promotor verknüpft ist;
ii) der Nucleotidsequenz, die mindestens 95 % Sequenzidentität mit SEQ ID NO: 17 umfasst, wobei die Nucleotidsequenz eine transkriptionelle regulatorische Aktivität aufweist, wobei die Enhancerdomäne sich in umgekehrter Orientierung befindet und funktionsfähig mit dem Promotor verknüpft ist; und
iii) einer Nucleotidsequenz, die mindestens 3 Kopien einer Sequenz umfasst, die 95 % Sequenzidentität mit SEQ ID NO: 1 aufweist, wobei die Nucleotidsequenz die transkriptionelle regulatorische Aktivität beibehält und wobei die Enhancerdomänen sich in umgekehrter Orientierung befinden und funktionsfähig mit dem Promotor verknüpft sind;
c) das Kontaktieren der Population der Zellen einer einkeimblättrigen Pflanze mit einer wirksamen Konzentration eines geeigneten Selektionsmittels; und
d) das Selektieren der Zelle der einkeimblättrigen Pflanze, die den selektierbaren Marker exprimiert, und dadurch das Identifizieren einer Zelle einer einkeimblättrigen Pflanze, die das Polynucleotid, das von Interesse ist, aufweist.

8. Verfahren nach Anspruch 7, wobei die Enhancerdomäne die in SEQ ID NO: 5 aufgeführte Sequenz nicht umfasst.

9. Verfahren nach Anspruch 7, wobei die Kopien des Enhancers direkt aneinander anliegen.

10. Verfahren nach Anspruch 7, 8 oder 9, wobei der selektierbare Marker für ein Polypeptid kodiert, das Toleranz für ein Herbizid verleiht.

11. Verfahren nach Anspruch 10, wobei das selektive Mittel ein Herbizid umfasst.

12. Verfahren nach Anspruch 7, wobei das DNA-Konstrukt in der 5'- bis 3'- oder 3'-bis 5'-Orientierung Folgendes umfasst: das erste Polynucleotid, das von Interesse ist, funktionsfähig mit einem zweiten heterologen Promotor verknüpft, der funktionsfähig mit mindestens einer der Enhancerdomänen verknüpft ist, die funktionsfähig mit dem ersten heterologen Promotor verknüpft sind, der funktionsfähig mit dem selektierbaren Marker verknüpft ist, wobei das erste Polynucleotid, das von Interesse ist, und der selektierbare Marker in von einander abweichenden Richtungen exprimiert werden.

13. Polynucleotid umfassend ein chimäres transkriptionelles regulatorisches Element, das einen Promotor umfasst, der die Expression in einer Zelle steuert, die mit mindestens einer Kopie einer heterologen Enhancerdomäne funktionsfähig verknüpft ist, wobei die Enhancerdomäne aus der Gruppe ausgewählt ist bestehend aus:
(a) der Nucleotidsequenz, die SEQ ID NO: 17 oder mindestens 3 Kopien von SEQ ID NO. 1 umfasst, wobei die Enhancerdomäne sich in umgekehrter Orientierung befindet und funktionsfähig mit dem Promotor verknüpft ist;
(b) der Nucleotidsequenz, die mindestens 95 % Sequenzidentität mit SEQ ID NO: 17 umfasst, wobei die Nucleotidsequenz eine regulatorische transkriptionelle Aktivität aufweist und sich in umgekehrter Orientierung befindet und funktionsfähig mit dem Promotor verknüpft ist; oder
(c) einer Nucleotidsequenz, die mindestens 3 Kopien einer Sequenz umfasst, die 95 % Sequenzidentität mit SEQ ID NO: 1 aufweist, wobei die Nucleotidsequenz eine transkriptionelle regulatorische Aktivität beibehält, wobei die Enhancerdomänen sich in umgekehrter Orientierung befinden und funktionsfähig mit dem Promotor verknüpft sind.

14. Polynucleotid nach Anspruch 13, wobei die Enhancerdomäne die Sequenz von SEQ ID NO: 5 nicht umfasst.

15. Polynucleotid nach Anspruch 13, wobei die Kopien des Enhancers direkt aneinander anliegen.

16. Expressionsvektor, Zelle einer einkeimblättrigen Pflanze oder einkeimblättrige Pflanze umfassend das Polynucleotid nach einem der Ansprüche 13 bis 15.

17. Verfahren nach einem der Ansprüche 1 bis 6 oder 7 bis 12, wobei die einkeimblättrige Pflanze Mais, Weizen, Reis, Gerste, Sorghumhirse oder Roggen ist.

18. Verfahren nach einem der Ansprüche 1-12 oder 17, wobei das Verfahren das Auftreten einer Einzelkopieintegration in die Zelle der einkeimblättrigen Pflanze erhöht.

## Revendications

1. Procédé d'expression d'un polynucléotide d'intérêt comprenant
l'introduction dans une cellule végétale de monocotylédone d'au moins une construction d'ADN comprenant une région chimère de régulation de la transcription comprenant au moins un domaine activateur fonctionnellement lié à un promoteur hétérologue, ladite région chimère de régulation de la transcription étant fonctionnellement liée au polynucléotide d'intérêt, ledit domaine activateur étant choisi parmi le groupe constitué de :
(a) la séquence nucléotidique comprenant SEQ ID NO: 17 ou au moins 3 copies de SEQ ID NO: 1, ledit domaine activateur se trouvant dans le sens inverse et étant fonctionnellement lié audit promoteur ;
(b) la séquence nucléotidique comprenant au moins 95 % d'identité de séquence par rapport à SEQ ID NO: 17, ladite séquence nucléotidique ayant une activité de régulation de la transcription et ledit domaine activateur se trouvant dans le sens inverse et étant fonctionnellement lié audit promoteur, ou
(c) une séquence nucléotidique comprenant au moins 3 copies d'une séquence ayant 95 % d'identité de séquence par rapport à SEQ ID NO: 1, ladite séquence nucléotidique conservant l'activité de régulation de la transcription, lesdits domaines activateurs se trouvant dans le sens inverse et étant fonctionnellement liés audit promoteur.

2. Procédé selon la revendication 1, ledit domaine activateur ne comprenant pas la séquence énoncée dans SEQ ID NO: 5.

3. Procédé selon la revendication 1, lesdites copies dudit activateur étant immédiatement adjacentes l'une par rapport à l'autre.

4. Procédé selon la revendication 1, 2 ou 3, ledit polynucléotide d'intérêt codant pour
(a) un polypeptide ; et/ou
(b) un marqueur pouvant être sélectionné.

5. Procédé selon la revendication 4, ledit marqueur pouvant être sélectionné comprenant un polynucléotide qui confère la tolérance à un herbicide.

6. Procédé selon la revendication 5, ledit procédé comprenant en outre la culture de ladite cellule végétale monocotylédone dans un milieu comprenant une concentration efficace de l'herbicide.

7. Procédé de sélection d'une cellule végétale de monocotylédone ayant un polynucléotide d'intérêt comprenant
(a) la fourniture d'une population de cellules végétales de monocotylédone ;
(b) l'introduction dans au moins l'une des cellules végétales de monocotylédone provenant de ladite population d'une construction d'ADN comprenant le polynucléotide d'intérêt et comprenant en outre une région chimère de régulation de la transcription comprenant au moins un domaine activateur fonctionnellement lié à un premier promoteur hétérologue, ladite région chimère de régulation de la transcription étant fonctionnellement liée à un marqueur pouvant être sélectionné, ledit domaine activateur étant choisi parmi le groupe constitué de :
i) la séquence nucléotidique comprenant SEQ ID NO: 17 ou au moins 3 copies de SEQ ID NO: 1, ledit domaine activateur se trouvant dans le sens inverse et étant fonctionnellement lié audit promoteur ;
ii) la séquence nucléotidique comprenant au moins 95 % d'identité de séquence par rapport à SEQ ID NO: 17, ladite séquence nucléotidique ayant une activité de régulation de la transcription, ledit domaine activateur se trouvant dans le sens inverse et étant fonctionnellement lié audit promoteur, et,
iii) une séquence nucléotidique comprenant au moins 3 copies d'une séquence ayant 95 % d'identité de séquence par rapport à SEQ ID NO: 1, ladite séquence nucléotidique conservant l'activité de régulation de la transcription, et lesdits domaines activateurs se trouvant dans le sens inverse et étant fonctionnellement liés audit promoteur ;
c) la mise en contact de ladite population de cellules végétales de monocotylédone avec une concentration efficace d'un agent de sélection approprié ; et,
d) la sélection de la cellule végétale monocotylédone exprimant ledit marqueur pouvant être sélectionné, et de là l'identification d'une cellule végétale monocotylédone ayant le polynucléotide d'intérêt.

8. Procédé selon la revendication 7, ledit domaine activateur ne comprenant pas la séquence énoncée dans le SEQ ID NO: 5.

9. Procédé selon la revendication 7, les copies dudit activateur étant immédiatement adjacentes l'une par rapport à l'autre.

10. Procédé selon la revendication 7, 8 ou 9, ledit marqueur pouvant être sélectionné codant pour un polypeptide qui confère la tolérance à un herbicide.

11. Procédé selon la revendication 10, ledit agent sélectif comprenant un herbicide.

12. Procédé selon la revendication 7, ladite construction d'ADN comprenant dans le sens 5' à 3' ou 3' à 5' : le premier polynucléotide d'intérêt fonctionnellement lié à un second promoteur hétérologue, fonctionnellement lié à au moins l'un desdits domaines activateurs, fonctionnellement lié au premier promoteur hétérologue, fonctionnellement lié au marqueur pouvant être sélectionné, ledit premier polynucléotidique d'intérêt et ledit marqueur pouvant être sélectionné étant exprimés dans des sens divergents.

13. Polynucléotide comprenant un élément chimère de régulation de la transcription comprenant un promoteur qui dirige l'expression d'une cellule fonctionnellement liée vers au moins une copie d'un domaine activateur hétérologue dans lequel ledit domaine activateur est choisi parmi le groupe constitué de :
(a) la séquence nucléotidique comprenant SEQ ID NO: 17 ou au moins 3 copies de SEQ ID NO: 1, ledit domaine activateur se trouvant dans le sens inverse et étant fonctionnellement lié audit promoteur ;
(b) la séquence nucléotidique comprenant au moins 95 % d'identité de séquence par rapport à SEQ ID NO: 17, ladite séquence nucléotidique ayant une activité de régulation de la transcription, et se trouvant dans le sens inverse et étant fonctionnellement liée audit promoteur ; ou,
(c) une séquence nucléotidique comprenant au moins 3 copies d'une séquence ayant 95 % d'identité de séquence par rapport à SEQ ID NO: 1, ladite séquence nucléotidique conservant l'activité de la régulation de la transcription, lesdits domaines activateurs se trouvant dans le sens inverse et étant fonctionnellement liés audit promoteur.

14. Polynucléotide selon la revendication 13, ledit domaine activateur ne comprenant pas la séquence de SEQ ID NO: 5.

15. Polynucléotide selon la revendication 13, les copies dudit activateur étant immédiatement adjacentes l'une par rapport à l'autre.

16. Vecteur d'expression, cellule végétale de monocotylédone ou plante monocotylédone comprenant le polynucléotide selon l'une quelconque des revendications 13 à 15.

17. Procédé selon l'une quelconque des revendications 1 à 6 ou 7 à 12, ladite monocotylédone étant le maïs, le blé, le riz, l'orge, le sorgho ou le seigle.

18. Procédé selon l'une quelconque des revendications 1 à 12 ou 17, ledit procédé accroissant un événement d'intégration de copie unique dans la cellule végétale de monocotylédone.
